(19) Europäisches Patentamt
European Patent Office
Office européen des brevets

(11) EP 3 545 967 A1

(12) # EUROPEAN PATENT APPLICATION

(43) Date of publication:
**02.10.2019 Bulletin 2019/40**

(21) Application number: **18164740.5**

(22) Date of filing: **28.03.2018**

(51) Int Cl.:
***A61K 39/00*** *(2006.01)* ***C07K 14/74*** *(2006.01)*
***A61P 35/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Deutsches Krebsforschungszentrum Stiftung des Öffentlichen Rechts
69120 Heidelberg (DE)**

(72) Inventor: **The designation of the inventor has not yet been filed**

(74) Representative: **Kuttenkeuler, David
Boehmert & Boehmert
Anwaltspartnerschaft mbB
Pettenkoferstrasse 22
80336 München (DE)**

(54) ## CANCER IMMUNIZATION PLATFORM

(57) The present invention pertains to a novel cell based tumor vaccine platform. The invention provides a method for modifying antigen presenting cells (APCs) to present both MHC class I and/or MHC class II peptides in context of improved peptide presentation protein complexes in order to increase activation of a patient's immune response. In this invention, an MHC II mRNA dendritic cell based vaccine platform was developed to activate $CD4^+$ T cells in patients and to enhance the anti-tumor response. The invariant chain (Ii) was modified and the semi-peptide CLIP was replaced with an MHC II binding peptide sequences of tumor associated antigens. These chimeric MHC II constructs are presented by APCs and induce proliferation of tumor specific $CD4^+$ T cells. The invention provides the constructs, proteins, nucleic acids, recombinant cells, as well as medical applications of these products.

EP 3 545 967 A1

SEQUENCE LISTING

<110>  Deutsches Krebsforschungszentrum Stiftung des oeffentlichen Rechts

<120>  CANCER IMMUNIZATION PLATFORM

<130>  D31591EP

<160>  29

<170>  PatentIn version 3.5

<210>  1
<211>  275
<212>  PRT
<213>  mus musculus

<400>  1

Met Asp Asp Gln Arg Asp Leu Ile Ser Asn His Glu Gln Leu Pro Ile
1               5                   10                  15

Leu Gly Asn Arg Pro Arg Glu Pro Glu Arg Cys Ser Arg Gly Ala Leu
            20                  25                  30

Tyr Thr Gly Val Ser Val Leu Val Ala Leu Leu Leu Ala Gly Gln Ala
        35                  40                  45

Thr Thr Ala Tyr Phe Leu Tyr Gln Gln Gln Gly Arg Leu Asp Lys Leu
    50                  55                  60

Thr Ile Thr Ser Gln Asn Leu Gln Leu Glu Ser Leu Arg Met Lys Asn
65                  70                  75                  80

Cys Gly Asn Cys Lys Phe Gly Phe Gly Gly Pro Asn Cys Thr Glu Lys
                85                  90                  95

Arg Val Met Asp Asn Met Leu Leu Gly Pro Val Lys Asn Val Thr Lys
            100                 105                 110

Tyr Gly Asn Met Thr Gln Asp His Val Met His Leu Leu Thr Arg Ser
        115                 120                 125

Gly Pro Leu Glu Tyr Pro Gln Leu Lys Gly Thr Phe Pro Glu Asn Leu
    130                 135                 140

Lys His Leu Lys Asn Ser Met Asp Gly Val Asn Trp Lys Ile Phe Glu
145                 150                 155                 160

Ser Trp Met Lys Gln Trp Leu Leu Phe Glu Met Ser Lys Asn Ser Leu
                165                 170                 175

```
Glu Glu Lys Lys Pro Thr Glu Ala Pro Pro Lys Val Leu Thr Lys Cys
            180                 185                 190

Gln Glu Glu Val Ser His Ile Pro Ala Val Tyr Pro Gly Ala Phe Arg
        195                 200                 205

Pro Lys Cys Asp Glu Asn Gly Asn Tyr Leu Pro Leu Gln Cys His Gly
    210                 215                 220

Ser Thr Gly Tyr Cys Trp Cys Val Phe Pro Asn Gly Thr Glu Val Pro
225                 230                 235                 240

His Thr Lys Ser Arg Gly Arg His Asn Cys Ser Glu Pro Leu Asp Met
                245                 250                 255

Glu Asp Leu Ser Ser Gly Leu Gly Val Thr Arg Gln Glu Leu Gly Gln
            260                 265                 270

Val Thr Leu
        275


<210>  2
<211>  277
<212>  PRT
<213>  Homo sapiens

<400>  2

Met His Arg Arg Arg Ser Arg Ser Cys Arg Glu Asp Gln Lys Pro Val
1               5                   10                  15

Met Asp Asp Gln Arg Asp Leu Ile Ser Asn Asn Glu Gln Leu Pro Met
            20                  25                  30

Leu Gly Arg Arg Pro Gly Ala Pro Glu Ser Lys Cys Ser Arg Gly Ala
        35                  40                  45

Leu Tyr Thr Gly Phe Ser Ile Leu Val Thr Leu Leu Leu Ala Gly Gln
    50                  55                  60

Ala Thr Thr Ala Tyr Phe Leu Tyr Gln Gln Gln Gly Arg Leu Asp Lys
65                  70                  75                  80

Asn Cys Gly Asn Cys Lys Phe Gly Phe Trp Gly Pro Asn Cys Thr Glu
                85                  90                  95

Arg Arg Leu Pro Met Gly Ala Leu Pro Gln Gly Pro Met Gln Asn Ala
            100                 105                 110
```

```
Thr Lys Tyr Gly Asn Met Thr Glu Asp His Val Met His Leu Leu Gln
        115                 120                 125

Asn Ala Asp Pro Leu Lys Val Tyr Pro Pro Leu Lys Gly Ser Phe Pro
    130                 135                 140

Glu Asn Leu Arg His Leu Lys Asn Thr Met Glu Thr Ile Asp Trp Lys
145                 150                 155                 160

Val Phe Glu Ser Trp Met His His Trp Leu Leu Phe Glu Met Ser Arg
                165                 170                 175

His Ser Leu Glu Gln Lys Pro Thr Asp Ala Pro Pro Lys Val Leu Thr
            180                 185                 190

Lys Cys Gln Glu Glu Val Ser His Ile Pro Ala Val His Pro Gly Ser
        195                 200                 205

Phe Arg Pro Lys Cys Asp Glu Asn Gly Asn Tyr Leu Pro Leu Gln Cys
    210                 215                 220

Tyr Gly Ser Ile Gly Tyr Cys Trp Cys Val Phe Pro Asn Gly Thr Glu
225                 230                 235                 240

Val Pro Asn Thr Arg Ser Arg Gly His His Asn Cys Ser Glu Ser Leu
                245                 250                 255

Glu Leu Glu Asp Pro Ser Ser Gly Leu Gly Val Thr Lys Gln Asp Leu
            260                 265                 270

Gly Pro Val Pro Met
        275


<210>  3
<211>  18
<212>  PRT
<213>  artificial

<220>
<223>  MHC peptide

<400>  3

Gly Thr Cys Arg Pro Gly Trp Arg Gly Ala Ala Cys Asn Gln Lys Ile
1               5                   10                  15

Leu Thr
```

<210> 4
<211> 20
<212> PRT
<213> artificial

<220>
<223> MHC peptide

<400> 4

Cys Gly Thr Cys Arg Pro Gly Trp Arg Gly Ala Ala Cys Asn Gln Lys
1 5 10 15

Ile Leu Thr Val
20

<210> 5
<211> 20
<212> PRT
<213> artificial

<220>
<223> MHC peptide

<400> 5

Arg His Arg Pro Leu Leu Glu Val Tyr Pro Glu Ala Asn Ala Pro Ile
1 5 10 15

Gly His Asn Arg
20

<210> 6
<211> 19
<212> PRT
<213> artificial

<220>
<223> MHC peptide

<400> 6

Asn Cys Gly Asn Cys Lys Phe Gly Phe Gly Gly Pro Asn Cys Thr Glu
1 5 10 15

Lys Arg Val

<210> 7
<211> 9
<212> PRT
<213> artificial

<220>
<223> MHC peptide

<400> 7

Thr Ala Pro Asp Asn Leu Gly Tyr Met
1               5

<210>  8
<211>  9
<212>  PRT
<213>  artificial

<220>
<223>  MHC peptide

<400>  8

Ala Ala Pro Asp Asn Leu Gly Tyr Met
1               5

<210>  9
<211>  9
<212>  PRT
<213>  artificial

<220>
<223>  MHC peptide

<400>  9

Thr Ala Pro Asp Asn Leu Gly Tyr Ala
1               5

<210>  10
<211>  9
<212>  PRT
<213>  artificial

<220>
<223>  MHC peptide

<400>  10

Ser Ser Met His Asn Ala Leu His Ile
1               5

<210>  11
<211>  9
<212>  PRT
<213>  artificial

<220>
<223>  MHC peptide

<400>  11

Arg His Pro Glu Asn Pro Asn Leu Leu
1               5

<210>  12
<211>  9

<212> PRT
<213> artificial

<220>
<223> MHC peptide

<400> 12

Leu Ile Arg Arg Asn Ile Phe Asp Leu
1 5

<210> 13
<211> 9
<212> PRT
<213> artificial

<220>
<223> MHC peptide

<400> 13

Leu Ile Arg Arg Asn Ile Phe Asp Leu
1 5

<210> 14
<211> 8
<212> PRT
<213> artificial

<220>
<223> MHC peptide

<400> 14

Lys Phe Phe Ser Tyr Leu Thr Leu
1 5

<210> 15
<211> 22
<212> PRT
<213> mus musculus

<400> 15

Leu Pro Lys Ser Ala Lys Pro Val Ser Gln Met Arg Met Ala Thr Pro
1 5 10 15

Leu Leu Met Arg Pro Met
20

<210> 16
<211> 22
<212> PRT
<213> homo sapiens

<400> 16

Leu Pro Lys Pro Pro Lys Pro Val Ser Lys Met Arg Met Ala Thr Pro

1 5 10 15

Leu Leu Met Gln Ala Leu
20

<210> 17
<211> 17
<212> PRT
<213> artificial

<220>
<223> MHC peptide

<400> 17

Ile Ser Gln Ala Val His Ala Ala His Ala Glu Ile Asn Glu Ala Gly
1 5 10 15

Arg

<210> 18
<211> 77
<212> DNA
<213> artificial

<220>
<223> primer

<400> 18
tgtctcactg accggcttgt atgctgccgc ccccgacaac ctgggctaca tgggaggtgg 60

cggatccgga ggtggtt 77

<210> 19
<211> 77
<212> DNA
<213> artificial

<220>
<223> primer

<400> 19
tgtctcactg accggcttgt atgctgccgc ccccgacaac ctgggctaca tgggaggtgg 60

cggatccgga ggtggtt 77

<210> 20
<211> 104
<212> DNA
<213> artificial

<220>
<223> primer

<400> 20
actggagagc cttcgcatga agcttggcac ctgcaggccc ggctggaggg gcgccgcctg 60

caaccagaag atcctgacca tggataacat gctccttggg cctg 104

<210> 21
<211> 77
<212> DNA
<213> artificial

<220>
<223> primer

<400> 21
tgtctcactg accggcttgt atgctagcag catgcacaac gccctgcaca tcggaggtgg 60

cggatccgga ggtggtt 77

<210> 22
<211> 77
<212> DNA
<213> artificial

<220>
<223> primer

<400> 22
tgtctcactg accggcttgt atgctagcag catgcacaac gccctgcaca tcggaggtgg 60

cggatccgga ggtggtt 77

<210> 23
<211> 107
<212> DNA
<213> artificial

<220>
<223> primer

<400> 23
actggagagc cttcgcatga agcttaactg cggcaactgc aagttcggct tcggcggccc 60

caactgcacc gagaagaggg tgatggataa catgctcctt gggcctg 107

<210> 24
<211> 77
<212> DNA
<213> artificial

<220>
<223> primer

<400> 24
aaccacctcc ggatccgcca cctcccatgt agcccaggtt gtcgggggcg gcagcataca 60

agccggtcag tgagaca 77

<210> 25
<211> 77
<212> DNA

<213> artificial

<220>
<223> primer

<400> 25
aaccacctcc ggatccgcca cctcccatgt agcccaggtt gtcgggggcg gcagcataca 60

agccggtcag tgagaca 77

<210> 26
<211> 104
<212> DNA
<213> artificial

<220>
<223> primer

<400> 26
caggcccaag gagcatgtta tccatggtca ggatcttctg gttgcaggcg gcgcccctcc 60

agccgggcct gcaggtgcca agcttcatgc gaaggctctc cagt 104

<210> 27
<211> 77
<212> DNA
<213> artificial

<220>
<223> primer

<400> 27
aaccacctcc ggatccgcca cctccgatgt gcagggcgtt gtgcatgctg ctagcataca 60

agccggtcag tgagaca 77

<210> 28
<211> 77
<212> DNA
<213> artificial

<220>
<223> primer

<400> 28
aaccacctcc ggatccgcca cctccgatgt gcagggcgtt gtgcatgctg ctagcataca 60

agccggtcag tgagaca 77

<210> 29
<211> 107
<212> DNA
<213> artificial

<220>
<223> primer

<400> 29
caggcccaag gagcatgtta tccatcaccc tcttctcggt gcagttgggg ccgccgaagc 60

```
cgaacttgca gttgccgcag ttaagcttca tgcgaaggct ctccagt                107
```

## Description

FIELD OF THE INVENTION

The present invention pertains to a novel cell based tumor vaccine platform. The invention provides a method for modifying antigen presenting cells (APCs) to present both Major Histocompatibility Complex (MHC) class I and/or MHC class II peptides in context of improved peptide presentation protein complexes in order to increase activation of a patient's immune response. In this invention, an MHC II mRNA dendritic cell based vaccine platform was developed to activate $CD4^+$ T lymphocytes (T cells) in patients and to enhance the anti-tumor response. The invariant chain (Ii) was modified and the semi-peptide CLIP was replaced with an MHC II binding peptide sequences of tumor associated antigens. These chimeric MHC II constructs are presented by APCs and induce proliferation of tumor specific $CD4^+$ T cells. The invention provides the constructs, proteins, nucleic acids, recombinant cells, as well as medical applications of these products.

DESCRIPTION

Metastatic melanoma is one of the most aggressive and lethal malignancies. Once it spreads to distant organs such as the brain, lungs or liver, the prognosis of melanoma is poor with a median survival of less than a year.

In the past few years, we are witnessing a renaissance era in the field of immunotherapy of metastatic melanoma. The FDA has recently approved new melanoma drugs based on the blockade of negative immune checkpoint. These treatments have shown a significant efficacy in a subset of melanoma patients. By blocking inhibitory signals, this therapeutic strategy allows the naturally occurring specific anti-melanoma cytotoxic T lymphocytes (CTLs) to execute their immune function and to eradicate the tumor. However, its effective use has multiple challenges. Mechanisms dealing with the resistance to targeted therapy, the tumor heterogeneity, genetic instability and transcriptional plasticity represent a major obstacle to effective treatment, and additional strategies will likely be required to enhance and broaden the anti-tumor activity.

Spontaneous anti-melanoma immune responses could be directed against melanocyte differentiation antigens (e.g., gp100, tyrosinase (Tyr), tyrosinase related proteins (TYRP1) and TYRP2) (7). Thus, several therapeutic strategies, aiming at enhancing the cellular anti-tumor immunity against these specific melanoma-associated antigens (MAAs) were developed. One such strategy uses autologous dendritic cells (DCs) loaded with MAAs. These DCs are extremely efficient in activating $CD8^+$ CTLs upon an antigen presentation. In addition to $CD8^+$ T cells, $CD4^+$ T cells were also shown to exert a role both in the induction and the effector phases of the anti-tumor response. Priming of $CD8^+$ CTLs requires full activation of DCs that is provided by $CD4^+$ T cells. In the effector phase, $CD4^+$ T cells recruited to the tumor site may exert their functions both directly on MHC II expressing tumor cells and indirectly by releasing cytokines that in turn activate anti-tumor functions of immune cells. Based on these principles, manipulation of DCs has also been assessed as a therapeutic approach for melanoma.

Anti-tumor vaccines find their application in many therapeutic fields ranging from anticancer treatments to treatment or prophylaxis of malignancies such as virally induced malignancies, but also sporadic malignancies that display tumor antigens such as MAGE, BAGE, RAGE, GAGE, SSX- 2, NY-ESO-I, CT-antigen, CEA, PSA, p53 or Tyrosinase or TYRP. The most preferred immune response to be obtained by any anti-tumor peptide vaccine is a T cell response, elicited by T cell epitopes within the peptides. There are two classes of MHC-molecules: MHC class I molecules that can be found on most cells having a nucleus which present peptides that result from proteolytic cleavage of mainly endogenous, cytosolic or nuclear proteins, and larger peptides. However, peptides derived from endosomal compartments or exogenous sources are also frequently found on MHC class I molecules. This non-classical way of class I presentation is referred to as cross- presentation in literature. MHC class II molecules can be found predominantly on professional APCs, and present predominantly peptides of exogenous proteins that are taken up by APCs during the course of endocytosis, and are subsequently processed. As for class I, alternative ways of antigen processing are described that allow peptides from endogenous sources to be presented by MHC class II molecules (e.g. autophagocytosis). Complexes of peptide and MHC class I molecule are recognized by CD8- positive cytotoxic T-lymphocytes bearing the appropriate TCR, whereas complexes of peptide and MHC class II molecule are recognized by CD4-positive helper T-cells bearing the appropriate TCR. A successful natural anti-tumor T-cell response should consist of both an HLA class I restricted CTL response and simultaneously an HLA class II restricted Th response, and may be advantageously accompanied by a B-cell response. Several publications have demonstrated that $CD4^+$ T -cells upon interaction with class II epitope presenting DC upregulate CD40 ligand.

Previously a novel genetic platform was developed which induces specific $CD8^+$ cytotoxic immune response by DCs vaccination against melanoma (Cafri G et al. Ann N Y Acad Sci 2013; 1283:87-90). The technology includes conversion of the MHC I light chain into an integral membrane protein by linking antigenic peptides to its N-terminus and the intracellular toll-like receptor (TLR4) signaling domain to its C-terminus (Cafri G et al. 2011 Int Immunol;

23:453-61). It was shown that efficient peptide presentation can be coupled to constitutive TLR4 signaling through the polypeptide product of a single gene, and that this dual effect can be achieved by virtue of mRNA electroporation. This modality was highly efficient in inhibiting tumor growth and improving survival, both in transplantable and spontaneous melanoma mouse models (Sharbi-Yunger A et al., Oncoimmunology. 2016).

Still, alternative effective treatment vaccination approaches for cancer therapy are desperately needed, in particular for aggressive tumors such as many forms of melanoma. Hence, the present invention seeks to provide a novel approach to treat cancerous disorders based on immunization of a patient with modified antigen presenting cells.

In a first aspect the above problem is solved by a method for the production of a cellular tumor-vaccine composition, the method comprising the steps of:

(a) Providing APCs,
(b) Introducing into said APCs one or more genetic expression constructs selected from:

 (i) A MHC class I fusion protein comprising an N-terminal MHC class I antigenic peptide, a β2 microglobulin (β2m), and a C-terminal membrane anchor, and
 (ii) A chimeric invariant chain (li), wherein the CLIP sequence is replaced by a sequence comprising an MHC class II antigenic peptide;

(c) Expressing the selected genetic expression constructs of (b) in said APCs, and
(d) Harvesting said APCs of (c) to obtain the cellular tumor-vaccination composition.

As used herein the term "cellular vaccine composition" or a "cellular tumor-vaccine composition" is a cell based composition comprising one or more species of cells as active ingredient which is suitable for administration to a mammal and which is capable of eliciting a specific immune response against a proliferative disease such as cancer. The cells comprised in the cellular vaccine composition of the invention are preferably antigen presenting cells.

In preferred embodiments of the invention an "antigen-presenting cell", abbreviated APC, is any of a variety of cells capable of acquiring, processing, presenting, or displaying at least one antigen or antigenic fragment on (or at) its cell surface. In general, the term "antigen-presenting cell" can be any cell that accomplishes the goal of the invention by aiding the enhancement of an immune response (i.e., from the T- cell or -B-cell arms of the immune system) against an antigen or antigenic composition. Such cells can be defined by those of skill in the art, using methods disclosed herein and in the art. As is understood by one of ordinary skill in the art (See for example Kuby, 2000, Immunology, 4th edition, W.H. Freeman and company), and used herein in certain embodiments, a cell that displays or presents an antigen normally or preferentially with a class II major histocompatibility molecule or complex to an immune cell is an APC. In certain aspects, a cell (e.g., an antigen-presenting cell) may be fused with another cell, such as a recombinant cell or a tumor cell that expresses the desired antigen. Methods for preparing a fusion of two or more cells is well known in the art, such as for example, the methods disclosed in Coding, J. W., Monoclonal Antibodies: Principles and Practice, pp. 65-66, 71-74 (Academic Press, 1986); Campbell, in: Monoclonal Antibody Technology, Laboratory Techniques in Biochemistry and Molecular Biology, Vol. 13, Burden & Von Knippenberg, Amsterdam, Elseview, pp. 75-83, 1984; Kohler & Milstein, Nature, 256:495-497, 1975; Kohler & Milstein, Eur. J. Immunol. , 6:511-519, 1976, Gefter et al, Somatic Cell Genet, 3:231-236,1977, each incorporated herein by reference. In some cases, the immune cell to which an antigen-presenting cell displays or presents an antigen to is a $CD4^+$ Th cell. Additional molecules expressed on the antigen-presenting cells or other immune cells may aid or improve the enhancement of an immune response. Secreted or soluble molecules, such as for example, cytokines and adjuvants, may also aid or enhance the immune response against an antigen. Such molecules are well known to one of skill in the art, and various examples are described herein.

In an exemplary, but preferred embodiment, an antigen-presenting cell is selected from a bone marrow dendritic cell (BMDC), B cell, dendritic cell, macrophage, activated epithelial cell, fibroblast, thymic epithelial cell, thyroid epithelial cell, glial cell, pancreatic beta cell, and a vascular endothelial cell. However, preferred embodiments of the invention pertain to a dendritic cell as APC. As used herein, dendritic cell (DC) may refer to any member of a diverse population of morphologically similar cell types found in lymphoid or non-lymphoid tissues. DCs may include, for example, "professional" antigen presenting cells, and have a high capacity for sensitizing MHC-restricted T cells. DCs may be recognized, for example, by function, by phenotype and/or by gene expression pattern, particularly by cell surface phenotype. These cells can be characterized by their distinctive morphology, high levels of surface MHC class II expression and ability to present antigen to $CD4^+$ and/or $CD8^+$ T cells, particularly to naive T cells (Steinman et al. (1991) Ann. Rev. Immunol. 9:271; incorporated herein by reference for its description of such cells).

In some embodiments, the APCs are autologous (i.e., derived from the subject to be treated). In other embodiments, APCs are obtained from a donor (i.e., allogeneic), for example, from a compatible donor, i.e., HLA typed so that they are histocompatible with the subject into which they will be transplanted.

The present invention includes the introduction of genetic constructs into APCs. Genetic constructs shall comprise any nucleic acid based vector or plasmid useful for introducing foreign genetic information into APCs in order to express the encoded protein in said APC. Preferably the genetic construct is capable for expressing (i) and/or (ii) on the cellular surface of the APC in order to "present" - in the sense of display - the recited antigenic sequences to the host's/patient's immune system. As used herein, the term "genetic construct" or "genetic expression construct" is meant to refer to a nucleic acid molecule that comprises a nucleic acid sequence that encodes a protein operably linked to elements necessary for expression (transcription and/or translation) of the protein sequence. In some embodiments, the genetic construct is DNA or RNA such as mRNA, preferably a plasmid or genome of a viral vector. In some embodiments, the genetic construct is RNA, preferably a genome of a retroviral vector. A typical genetic expression construct comprises in addition to the sequence encoding the protein to be expressed a promoter element, sometimes enhancer elements.

Introducing a genetic expression construct of the invention into an APC may be performed with any method for cell transfection or transduction. Examples of transfecting or introducing antigen into the antigen-presenting cells include, for example, but are not limited to, electroporation, injection, sonication loading, liposome-mediated transfection, and receptor-mediated transfection, or viral based transduction. In a particular embodiment, the introduction of antigen into the antigen-presenting cells is performed by electroporation, such as the electroporation of an mRNA into an APC. Electroporation may involve the exposure of a suspension of cells and antigen to a high- voltage electric discharge. In some variants of this method, certain cell wall-degrading enzymes, such as pectin-degrading enzymes, are employed to render the target recipient cells more susceptible to transformation by electroporation than untreated cells. Electroporation methods are well known in the art, and the present invention shall not be restricted to any specific method for electroporating APCs. However, a preferred method is described in Cafri G et al. Mol Ther 2015: "mRNA-transfected dendritic cells expressing polypeptides which link MHC-I presentation to constitutive TLR4 activation confer tumor immunity". Hence in some preferred embodiments the genetic expression construct is an mRNA expression vector, and the introduction of said genetic expression constructs into said antigen presenting cells is performed by RNA electroporation.

A MHC class I fusion protein according to the invention preferably comprises an N-terminal MHC class I antigenic peptide, β2 microglobulin (β2m), and a C-terminal membrane anchor. Preferably the MHC class I fusion protein according to the invention comprises in N- to C-terminal order the MHC class I antigenic peptide, the β2m, and the C-terminal membrane anchor. In addition thereto the C class I antigenic peptide and the β2m may be connected by a linker amino acid sequence. Further, the β2m and the C-terminal membrane anchor may be connected by a bridge sequence. The bridge sequence may include amino acid residues of the extracellular domain of the β2m and/or the membrane anchor.

The term "β2m" shall in context of the invention refer to a protein expressed by the human gene beta-2-microglobulin, annotated under HGNC:914 of the HUGO gene nomenclature in the version of the present priority date (https://www.genenames.org/cgi-bin/gene symbol report?hgnc id=HGNC:914). The protein information is derivable under the P61769 in the Uniprot database (http://www.uniprot.org/uniprot/P61769) also in the version of the present priority date. The term shall also encompass homologs and orthologs of the human protein, in particular mouse and rat homologs. All genes or proteins described herein are referred to by their HUGO gene nomenclature Committee annotation. Reference is made to Gray KA, Yates B, Seal RL, Wright MW, Bruford EA. genenames.org: the HGNC resources in 2015. Nucleic Acids Res. 2015 Jan;43(Database issue):D1079-85. doi: 10.1093/nar/gku1071. PMID:25361968. For the present purpose the database in the version of March, 2018 was used: HGNC Database, HUGO Gene Nomenclature Committee (HGNC), EMBL Outstation - Hinxton, European Bioinformatics Institute, Wellcome Trust Genome Campus, Hinxton, Cambridgeshire, CB10 1SD, UK (www.genenames.org).

A C-terminal membrane anchor is preferably selected from $K^b$ or a partial Toll like receptor (TLR) 4 and/or TLR2 protein comprising a transmembrane domain and/or a TLR4 intracellular signaling portion.

HLA class II histocompatibility antigen gamma chain also known as HLA-DR antigens-associated invariant chain or CD74 (Cluster of Differentiation 74), is a protein that in humans is encoded by the CD74 gene (HGNC:1697). The invariant chain (abbreviated "Ii") is a polypeptide involved in the formation and transport of MHC class II protein. The cell surface form of the invariant chain is known as CD74. A chimeric invariant chain (Ii) of the invention preferably comprises chimeric invariant chain (Ii) sequence, but wherein the Class II-associated invariant chain peptide (CLIP) sequence is replaced by a sequence comprising an MHC class II antigenic peptide.

In context of the invention a MHC class I antigenic peptide shall refer to a peptide sequence having the capability to bind and be presented by an MHC class I protein complex. Such peptides are well known in the art and preferably comprise, consist essentially of, or consist of, a sequence of 8, 9, 10 or 11 amino acids. Such class I peptide sequences are preferably derived from a tumor associated antigen or tumor specific antigen (TAA or TSA).

In context of the invention a MHC class II antigenic peptide shall refer to a peptide sequence having the capability to bind and be presented by an MHC class II protein complex. Such peptides are well known in the art and preferably comprise, consist essentially of, or consist of, a sequence of 8 to 30, preferably 10 to 25, more preferably 12 to 18 amino acids. Such class II peptide sequences are preferably derived from a tumor associated antigen or tumor specific antigen (TAA or TSA).

In some preferred embodiments the method of the invention is an *ex-vivo* or *in-vitro* method that is the method is preferably performed in cell culture.

A preferred embodiment pertains to the above described method, but wherein at least two different genetic expression constructs according to (i) are introduced into said APCs, with one genetic expression construct according to (i) comprising the $K^b$ as C-terminal membrane anchor, and the other genetic expression construct according to (i) comprising the partial TLR4 or TLR2 protein as C-terminal membrane anchor. Preferably the at least two different genetic expression constructs according to (i) are introduced into said APCs in a ration of bout 1:1.

The term "about" in context of the various aspects and embodiments of the invention shall indicate a variation of +/- 20% of the indicated value or values. In preferred embodiments the term "about" shall indicate a variation of +/- 15%, preferably +/- 10%, more preferably +/- 5%, and most preferably +/- 2% of the indicated value or values.

Another preferred embodiments of the invention further pertains to a method, wherein step (b) comprises introducing into said APCs one or more genetic expression constructs according to (i) and (ii), preferably simultaneously. In this way, a cellular tumor vaccine composition is provided eliciting both and simultaneously an HLA class I and II mediated immune response, which is surprisingly advantageous in context of the herein disclosed invention.

An antigenic peptide or antigenic peptide sequence according to the invention is preferably a peptide sequence associated with a tumor. In preferred embodiments the MHC class I antigenic peptide and the MHC class II antigenic peptide are selected from antigenic peptide sequences derived from TAA which are associated with the same tumor, such as melanoma. Even more preferably the MHC class I antigenic peptide and the MHC class II antigenic peptide are selected from antigenic peptide sequences of the same TAA.

According to the present invention, a "tumor-associated antigen" preferably comprises any antigen which is characteristic for tumors or cancers as well as for tumor or cancer cells with respect to type and/or expression level. In one embodiment , the term "tumor- associated antigen" relates to proteins that are under normal conditions, i.e. in a healthy subject, specifically expressed in lower amounts in healthy tissue, or in a limited number of organs and/or tissues or in specific developmental stages, for example, the tumor-associated antigen may be under normal conditions specifically expressed in stomach tissue, preferably in the gastric mucosa , in reproductive organs , e.g., in testis, in trophoblastic tissue, e.g., in placenta, or in germ line cells, and are expressed or aberrantly expressed in one or more tumor or cancer tissues. On the other hand a "tumor-specific antigen" shall refer to an antigenic compound that is specifically expressed in tumor cells, such as a mutated version of a protein.

A preferred TAA of the invention is selected from gp100 (HGNC:10880), tyrosinase (Tyr; HGNC:12442), tyrosinase related protein (TYRP)1 (HGNC:12450) or TYRP2 (HGNC:2709). Immunogenic peptide sequences of these TAA are well known in the art and may be used in context of the present invention.

In a preferred MHC class II antigenic peptide is selected from an MHC class II antigenic sequence derived from TYRP1 or Tyr.

According to a preferred embodiment of the method of the invention, the MHC class II antigenic peptide sequence is selected from a sequence according to any of SEQ ID NO: 3 to 6, preferably 5 or 6.

According to a preferred embodiment of the method of the invention, the MHC class I antigenic peptide is selected from a sequence according to any of SEQ ID NO: 7 to 14, preferably 10 to 14.

A preferred method of the invention pertains to a method wherein the genetic expression construct of step (b) (i) [MHC class I genetic constructs] comprises an antigenic peptide of Tyr and the genetic expression construct of step (b) (ii) [MHC class II genetic constructs] comprises an antigenic peptide sequence derived of TYRP1 or TYRP2. In another embodiment, the genetic expression construct of step (b)(i) [MHC class I genetic constructs] comprises an antigenic peptide of TYRP1 or TYRP2 and the genetic expression construct of step (b) (ii) comprises an antigenic peptide sequence derived of Tyr. In yet another embodiment, the method comprises the introduction of at least two genetic expression constructs according to step (b) (i), wherein the at least two genetic expression constructs comprise different antigenic peptide sequences, for example (1) wherein the at least two different antigenic peptide sequences in the genetic expression construct according to step (b) (i) are derived of the same TAA, for example of Tyr, or (2) wherein the at least two different antigenic peptide sequences in the genetic expression construct according to step (b) (i) are derived of two different TAA, for example of Tyr and TYRP1, or TYR and TYRP2,or TYRP1 and TYRP2. Yet another alternative or additional embodiment of the invention may include a method which comprises the introduction of at least two genetic expression constructs according to step (b)(ii) [MHC class II genetic constructs], wherein the at least two genetic expression constructs comprise different antigenic peptide sequences, for example (1) wherein the at least two different antigenic peptide sequences in the genetic expression construct according to step (b) (ii) are derived of the same TAA, for example of Tyr, or (2) wherein the at least two different antigenic peptide sequences in the genetic expression construct according to (ii) are derived of two different TAA, for example of Tyr and TYRP1, or TYR and TYRP2,or TYRP1 and TYRP2.

In some embodiments the method of the present invention may additionally include between steps (c) and step (d) an additional step where the APCs are cultured and/or expanded.

In some additional embodiments the disclosed method for the production of a cellular tumor-vaccine composition may be used in the context of infectious diseases or other proliferative disorders. In these aspects the described genetic expression constructs according to steps (b) (i)) and (ii)) comprise instead of a TAA or TSA derived antigenic peptide sequence, a peptide sequence specific for the causal agent of the infectious or proliferative disease, in order to allow the cellular composition to effectively prime a patient's immune system against the disease to be treated.

The invention in one additional aspect also pertains to a cellular composition obtainable by a method for the production of a cellular tumor-vaccine composition.

Another aspect of the present invention also pertains to a cell, comprising a genetic expression construct, or an expression product of a genetic expression construct according to step (b)(i) [MHC class I genetic constructs] and/or (ii) [MHC class II genetic constructs] according to the herein described method. Also provided are cell preparations of multiple cells produced according to the invention (harvested according to the invention).

In another aspect the invention provides a nucleic acid comprising a nucleotide sequence of a genetic expression constructs as described herein.

The products and compositions, in particular the genetic expression constructs and the cellular compositions of the invention, are preferably used for the treatment of diseases, are therefore useful and should be used in medicine. Hence provided is a product for use in medicine, wherein the product is a cellular vaccine composition, a cell according or a nucleic acid according to the invention.

As mentioned before, the methods and products of invention are for use in the treatment of a tumor disease, such as melanoma. However, as explained above, the methods and products may also be used in context of infectious diseases or other proliferative disorders.

A medical use in accordance with the herein described invention comprises preferably a step of administration of a cellular tumor-vaccine composition, or other inventive cellular compositions - for example for the treatment of infectious diseases - in a therapeutically effective amount to a patient in need of such a treatment.

In another aspect the present invention also pertains to an antigenic peptide, or immunogen, comprising, consisting essentially of, or consisting of, a sequence according to any of SEQ ID NO: 3 to 14, or a variant peptide or immunogen, comprising, consisting essentially of, or consisting of, a sequence according to any of SEQ ID NO: 3 to 14 with not more than 3, preferably not more than 2, more preferably not more than 1 amino acid substitution, deletion, addition or insertion, compared to these sequences in each case independently.

In some embodiments of the invention the antigenic peptide according of the invention consists essentially of, or consists of, no more than 200 amino acids, preferably no more than 100, 50 or most preferably 25 amino acids. The length of the peptide is dependent on whether it is to be presented in the context of MHC class I or class II. The person of skill is aware of these differences.

Preferably, if the antigenic peptide of the invention is a MHC class II peptide, the peptide comprises, consisting essentially of, or consisting of, a sequence according to any of SEQ ID NO: 3 to 6, more preferably 5 or 6, or a variant peptide or immunogen, comprising, consisting essentially of, or consisting of, a sequence according to any of SEQ ID NO: 3 to 6, more preferably 5 or 6, with not more than 3, preferably not more than 2, more preferably not more than 1 amino acid substitution, deletion, addition or insertion, compared to these sequences in each case independently.

Preferably, if the antigenic peptide of the invention is a MHC class I peptide, the peptide comprises, consisting essentially of, or consisting of, a sequence according to any of SEQ ID NO: 7 to 14, more preferably 10 to 14, or a variant peptide or immunogen, comprising, consisting essentially of, or consisting of, a sequence according to any of SEQ ID NO: 7 to 14, more preferably 10 to 14, with not more than 3, preferably not more than 2, more preferably not more than 1 amino acid substitution, deletion, addition or insertion, compared to these sequences in each case independently.

An MHC class I antigenic peptide according to the invention preferably elicits an MHC class I dependent immune response. Similarly, an MHC class II antigenic peptide according to the invention preferably elicits an MHC class II dependent immune response.

Furthermore provided is a chimeric CD74 protein, comprising a sequence wherein the MHC class II-associated invariant chain (Ii)-derived peptide (CLIP) is replaced by a sequence of an antigenic peptide for MHC class II according to the herein described invention. CLIP sequences are known in the art and are CLIP (81-102) mouse: LPKSAKPVSQMRMATPLLMRPM (SEQ ID NO: 15) and CLIP (81-102) human: LPKPPKPVSKMRMATPLLMQAL(SEQ ID NO: 16). CD74 mouse and human sequences are shown in SEQ ID NO: 1 and 2 respectively.

A chimeric CD74 protein of the invention, comprises a sequence where the native CLIP is replaced by an antigenic MHC class II peptide sequence, preferably selected from any one of SEQ ID NO: 3 to 6, more preferably SEQ ID NO: 5 or 6.

Also provided is a nucleic acid comprising a nucleotide sequence encoding for the antigenic peptide or the chimeric CD74 protein of the invention, preferably wherein the nucleic acid is an expression vector.

Another aspect pertains to a recombinant host cell comprising the antigenic peptide or the chimeric CD74 protein or the nucleic acid of the invention.

The here disclosed compounds and cells are products for use in medicine, preferably for use in the treatment of a tumor disease, preferably melanoma.

The present invention pertains in its various aspects and embodiments preferably to a use in or to a method for the treatment of cancer, or a tumor disease. In context of the invention the term "cancer" or "tumour" are used interchangingly, and refer to a cellular disorder characterized by uncontrolled or dysregulated cell proliferation, decreased cellular differentiation, inappropriate ability to invade surrounding tissue, and/or ability to establish new growth at ectopic sites. The term "cancer" includes, but is not limited to, solid tumors and blood-borne tumors. The term "cancer" encompasses diseases of skin, tissues, organs, bone, cartilage, blood, and vessels. The term "cancer" further encompasses primary and metastatic cancers. Non-limiting examples of solid tumors include pancreatic cancer; bladder cancer; colorectal cancer; breast cancer, including metastatic breast cancer; prostate cancer, including androgen-dependent and androgen-independent prostate cancer; renal cancer, including, e.g., metastatic renal cell carcinoma; hepatocellular cancer; lung cancer, including, e.g., non-small cell lung cancer (NSCLC), bronchioloalveolar carcinoma (BAC), and adenocarcinoma of the lung; ovarian cancer, including, e.g., progressive epithelial or primary peritoneal cancer; cervical cancer; gastric cancer; esophageal cancer; head and neck cancer, including, e.g., squamous cell carcinoma of the head and neck; skin cancer, including e.g., malignant melanoma; neuroendocrine cancer, including metastatic neuroendocrine tumors; brain tumors, including, e.g., glioma, anaplastic oligodendroglioma, adult glioblastoma multiforme, and adult anaplastic astrocytoma; bone cancer; soft tissue sarcoma; and thyroid carcinoma.

Non-limiting examples of hematologic malignancies include acute myeloid leukemia (AML); chronic myelogenous leukemia (CML), including accelerated CML and CML blast phase (CML-BP); acute lymphoblastic leukemia (ALL); chronic lymphocytic leukemia (CLL); Hodgkin's disease (HD); non-Hodgkin's lymphoma (NHL), including follicular lymphoma and mantle cell lymphoma; B-cell lymphoma; T-cell lymphoma; multiple myeloma (MM); Waldenstrom's macroglobulinemia; myelodysplastic syndromes (MDS), including refractory anemia (RA), refractory anemia with ringed siderblasts (RARS), (refractory anemia with excess blasts (RAEB), and RAEB in transformation (RAEB-T); and myeloproliferative syndromes.

In some embodiments, the examples of the cancer to be treated include, but are not limited to, lung cancer, head and neck cancer, colorectal cancer, pancreatic cancer, colon cancer, breast cancer, ovarian cancer, prostate cancer, stomach cancer, kidney cancer, liver cancer, brain cancer, bone cancer, and leukemia. In some embodiments, the examples of cancer to be treated are chosen from lung cancer, head and neck cancer, colorectal cancer, pharynx cancer, epidermoid cancer, and pancreatic cancer.

In some embodiments the preferred cancer is melanoma.

In yet another aspect the invention further pertains to pharmaceutical compositions of the various products of the invention, which are in the pharmaceutical composition formulated together with at least one pharmaceutically acceptable carrier and/or excipient. All components and products of the invention may be used in their described form or as any salt, solvate, derivative or isomer thereof.

The pharmaceutical compositions of the invention are preferably in a form for administration in various known manners, such as orally, parenterally, by inhalation spray, or via an implanted reservoir. The term "parenteral" as used herein includes subcutaneous, intracutaneous, intravenous, intramuscular, intraarticular, intraarterial, intrasynovial, intrasternal, intrathecal, intralesional and intracranial injection or infusion techniques.

An oral composition can be any orally acceptable dosage form including, but not limited to, tablets, capsules, emulsions and aqueous suspensions, dispersions and solutions. Commonly used carriers for tablets include lactose and corn starch. Lubricating agents, such as magnesium stearate, are also typically added to tablets. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A sterile injectable composition (e.g., aqueous or oleaginous suspension) can be formulated according to techniques known in the art using suitable dispersing or wetting agents (such as, for example, Tween 80) and suspending agents. The sterile injectable preparation can also be a sterile injectable solution or suspension in a non-toxic parenterally acceptable diluent or solvent, for example, as a solution in 1,3-butanediol. Among the pharmaceutically acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or di-glycerides). Fatty acids, such as oleic acid and its glyceride derivatives are useful in the preparation of injectables, as are natural pharmaceutically-acceptable oils, such as olive oil or castor oil, such as in their polyoxyethylated versions. These oil solutions or suspensions can also contain a long-chain alcohol diluent or dispersant, or carboxymethyl cellulose or similar dispersing agents.

An inhalation composition can be prepared according to techniques well known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art.

A topical composition can be formulated in form of oil, cream, lotion, ointment and the like. Suitable carriers for the composition include vegetable or mineral oils, white petrolatum (white soft paraffin), branched chain fats or oils, animal fats and high molecular weight alcohols (greater than C 12). In some embodiments, the pharmaceutically acceptable carrier is one in which the active ingredient is soluble. Emulsifiers, stabilizers, humectants and antioxidants may also be included as well as agents imparting color or fragrance, if desired. Additionally, transdermal penetration enhancers may be employed in these topical formulations. Examples of such enhancers can be found in U.S. Patents 3,989,816 and 4,444,762.

Creams may be formulated from a mixture of mineral oil, self-emulsifying beeswax and water in which mixture the active ingredient, dissolved in a small amount of an oil, such as almond oil, is admixed. An example of such a cream is one which includes about 40 parts water, about 20 parts beeswax, about 40 parts mineral oil and about 1 part almond oil. Ointments may be formulated by mixing a solution of the active ingredient in a vegetable oil, such as almond oil, with warm soft paraffin and allowing the mixture to cool. An example of such an ointment is one which includes about 30% by weight almond and about 70% by weight white soft paraffin.

A pharmaceutically acceptable carrier refers to a carrier that it is compatible with active ingredients of the composition (and in some embodiments, capable of stabilizing the active ingredients) and not deleterious to the subject to be treated. For example, solubilizing agents, such as cyclodextrins (which form specific, more soluble complexes with the at least one compound and/or at least one pharmaceutically acceptable salt described herein), can be utilized as pharmaceutical excipients for delivery of the active ingredients. Examples of other carriers include colloidal silicon dioxide, magnesium stearate, cellulose, sodium lauryl sulfate, and D&C Yellow # 10. Hydrophilic excipients such as synthetic and natural polymers (e.g. albumin and derivatives thereof), are also examples of pharmaceutically acceptable carriers.

The present invention will now be further described in the following examples with reference to the accompanying figures and sequences, nevertheless, without being limited thereto. For the purposes of the present invention, all references as cited herein are incorporated by reference in their entireties. In the Figures:

**Figure 1:** **Scheme of the construct** (A) Genetic design of the MHC-I β2m based bifunctional constructs. The sites of promoter (pr), leader peptide (lead), antigenic peptide (p), linker peptide (li) and bridge (br) are shown. (B) Anticipated configuration of the polypeptide products with a linked antigenic peptide in the context of an endogenous MHC-I heavy α chain. (C) Protein design of the chimeric Invariant chain. (D) Anticipated configuration of the peptide in the context of endogenous MHC-II heavy chains. (E) Designations of the different constructs generated and used for immunizations in this study. Figures

**Figure 2:** **Immunization with BMDCs electroporated with MHC-I constructs induce potent CTLs.** BMDCs were electroporated with 10μg of mRNA followed by 6hrs incubation. Cells (0.5x106) were injected i.p into naive mice, 3 times at weekly intervals. Ten days following last immunization killing assays were performed. (A) CTL *in vivo.* Peptide loaded target cells were injected into immunized mice. Assays were done 18hrs later. (B) CTL *in vitro.* Melanoma cell lines expressing MAAs or D122 Lewis lung carcinoma (negative control) served as target cells. Data are representative of two independent experiments.

**Figure 3:** **Invariant chain chimeric constructs induce proliferation of $CD4^+$ T cells.** (A) BMDCs, mature or immature, were electroporated with 10μg of transcribed mRNA of the OVA-CLIP construct and co-incubated with OT-II $CD4^+$ T cells. Proliferation was measured by 3H-Thymidine uptake. Peptide loaded BMDCs served as positive control whereas BMDCs electroporated with an irrelevant MHC-II construct served as negative control. (B) and (C) C57Bl/6 naive mice were immunized with a E122 or E130 emulsified in CFA. Eleven days following immunization, popliteal LNs were excised, $CD4^+$ T cells were isolated and co-incubated with BMDCs electroporated with 10μg of E122 or E130 transcribed mRNA respectively. Proliferation was measured by 3H-Thymidine uptake. Peptide loaded BMDCs served as positive control whereas BMDCs electroporated with an irrelevant MHC-II construct served as negative control. (D) C57Bl/6 naive mice were immunized with E122 or E130 in CFA. Eleven days following immunization, popliteal LNs whole cells were co-incubated for 72hrs with 4μg of E122 or E130 peptide respectively. Cells were then harvested and incubated with tetramers and anti CD4. Staining was quantitated flow cytometry. Data are representative of two independent experiments. *P<0.05, **P<0.01, ***P<0.001

**Figure 4:** **Immunization with BMDCs elctroporated with constructs inhibits tumor growth and improves survival of Ret tumor bearing mice.** (A) Experimental scheme. (B-J) On day 0, melanoma Ret cells, 1x105/mouse, were inoculated i.f.p into mice (n>8 per group). Three days later, BMDCs were electroporated with 10-20μg of transcribed mRNA (combinations of MHC-I or MHC-I and MHC-II or single construct) and injected i.p (0.5x106 /mouse), 3 times at weekly intervals. Tumor growth was monitored. Mice were sacrificed when tumors reached 8mm diameter. Survival curves were drawn. Data are representative of two independent experiments. *P<0.05, **P<0.01, ***P<0.001

**Figure 5:** **Immunization with BMDCs electroporated with MHC-I Construct induces IFNγ secreting CD8+ cells in Ret tumor bearing mice.** Tumor bearing mice (n=3) were immunized, 3 times at weekly intervals, with BMDCs electroporated with 10-20μg of transcribed mRNA. Spleens were harvested 10 days following the last immunization and stained for cell surface markers and intracellular cytokines. (A) E120+E124 TYRP1+Tyr MHC-I combination. (B) E120+E122-CLIP TYRP1 MHC-I+MHC-II combination. (C) E124+E130-CLIP Tyr MHC-I+MHC-II combination. *P<0.05, **P<0.01, ***P<0.001

**Figure 6:** **Immunization with BMDCs electroporated with MHC-I and MHC-II Construct combinations induces Th1 immune response in Ret tumor bearing mice.** Tumor bearing mice (n=3) were immunized, 3 times at weekly intervals, with BMDCs electroporated with 10-20μg of transcribed mRNA. Spleens were harvested 10 days following the last immunization and stained for cell surface markers and intracellular cytokines. (A) E120+E124 TYRP1+Tyr MHC-I combination. (B) E120+E122-CLIP TYRP1 MHC-I+MHC-II combination. (C) E124+E130-CLIP Tyr MHC-I+MHC-II combination. *P<0.05, **P<0.01, ***P<0.001

**Figure 7:** **Immunization with combinations of MHC-I and MHC-II Constructs induces specific CTLs and a Th2 immune response in Ret tumor bearing mice.** Tumor bearing mice (n=3) were immunized, 3 times at weekly intervals, with BMDCs electroporated with 10-20μg of transcribed mRNA. Spleens were harvested 10 days following the last immunization and tested for presence of CTLs by *in vivo* kill assay (A) and then stained for cell surface markers and intracellular cytokines (B). *P<0.05, **P<0.01, ***P<0.001

**Figure 8:** **Constructs immunization does not induce statistically significant levels of Tregs and MDSCs.** Mice (n=3) were immunized with BMDCs electroporated with 10-20μg transcribed mRNA 3 times at weekly intervals. Ten days following immunization, spleens were harvested and stained for immune profiling markers of MDSCs, Th17 and Tregs.

And in the sequences:
Sequence for murine CD74: NCBI Reference Sequence NM_001042605.1 (SEQ ID NO: 1). CLIP is shown in bold letters:

MDDQRDLISNHEQLPILGNRPREPERCSRGALYTGVSVLVALLLAGQATTAYFLYQQQGRLD
KLTITSQNLQLESLRMK-**NCGNCKFGFGGPNCTEKRV**-
MDNMLLGPVKNVTKYGNMTQDHVMHLLTRSGPLEYPQLKGTFPENLKHLKNSMDGVNW
KIFESWMKQWLLFEMSKNSLEEKKPTEAPPKVLTKCQEEVSHIPAVYPGAFRPKCDENGNY
LPLQCHGSTGYCWCVFPNGTEVPHTKSRGRHNCSEPLDMEDLSSGLGVTRQELGQVTL

Sequence for human CD74: NCBI Reference Sequence: NM_001025159.2 (SEQ ID NO: 2) CLIP is shown in bold letters:

MHRRRSRSCREDQKPVMDDQRDLISNNEQLPMLGRRPGAPESKCSRGALYTGFSILVTLLL
AGQATTAYFLYQQQGRLDK-**NCGNCKFGFWGPNCTERRL**-
PMGALPQGPMQNATKYGNMTEDHVMHLLQNADPLKVYPPLKGSFPENLRHLKNTMETID
WKVFESWMHHWLLFEMSRHSLEQKPTDAPPKVLTKCQEEVSHIPAVHPGSFRPKCDENGN
YLPLQCYGSIGYCWCVFPNGTEVPNTRSRGHHNCSESLELEDPSSGLGVTKQDLGPVPM

MHC class II peptides:

| | | |
|---|---|---|
| a) | **TYRP1 (111-128) GTCRPGWRGAACNQKILT | allele: H2-IAb (SEQ ID NO: 3) |
| b) | **TYRP1 (110-129) CGTCRPGWRGAACNQKILTV | allele: H2-IAb (SEQ ID NO: 4) |
| c) | TYR (403-422) RHRPLLEVYPEANAPIGHNR | allele: H2-IAb (SEQ ID NO: 5) |
| d) | TYR (99-117) NCGNCKFGFGGPNCTEKRV | allele: H2-IAb (SEQ ID NO: 6) |

**TYRP-1 (p113-126) published by Rausch et al, J Immunol 2010

MHC class I peptides:

| | | |
|---|---|---|
| a) | *TYRP-1 (455-463) -variant 1 TAPDNLGYM | allele: H2-Db (SEQ ID NO: 7) |
| b) | *TYRP-1 (455-463) -variant 2 AAPDNLGYM | allele: H2-Db (SEQ ID NO: 8) |
| c) | *TYRP-1 (455-463) -native TAPDNLGYA | allele: H2-Db (SEQ ID NO: 9) |
| d) | Tyr (360-368) SSMHNALHI | allele: H2-Db (SEQ ID NO: 10) |
| e) | Tyr (255-264) RHPENPNLL | allele: H2-Db (SEQ ID NO: 11) |
| f) | mTyr (118-126) LIRRNIFDL | allele: H2-Db (SEQ ID NO: 12) |
| g) | mTyr (445-452) LGYDYSYL | allele: H2-Kb (SEQ ID NO: 13) |
| h) | mTyr (133-140) KFFSYLTL | allele: H2-Kb (SEQ ID NO: 14) |

* Published by Dougan et al, Cancer Immunol Res 2013

EXAMPLES

**Materials and Methods**

Mice

C57BL/6 (H-2b), B6.SJL (CD45.1/H-2b) and OT-II $CD4^+$ T cell transgenic mice for the OVA323-339 MHC-II antigenic peptide were purchased from Harlan (Rehovot, Israel). Animals were maintained and treated according to the Weizmann Institute of Science and National Institute of Health guidelines.

Cell lines

F10.9-FD21, melanoma cell line transfected with H-2Db (19) was cultured in DMEM (Invitrogen) containing 10% FCS, 2mM L-glutamine, 1mM sodium pyruvate, 1% nonessential amino acids and combined antibiotics.

3LL-D122- Lewis Lung Carcinoma cells do not express melanoma associated antigens and serves as a negative control (20). Cells were cultured in DMEM (Invitrogen) with 10% FCS (Hyclone), 2mM L-glutamine, 1mM sodium pyruvate, and combined antibiotics.

Ret mouse melanoma cell line was previously established from the skin tumors of ret transgenic mice (21). Cells were cultured in RPMI 1640 (GibcoBRL) containing 10% FCS, 2mM L-glutamine, 1mM sodium pyruvate, 1% nonessential amino acids and combined antibiotics. In this study, ret mouse melanoma cell line was used to inoculate a tumor intra-foodpad in C57BL/6 mice.

Antibodies

The anti-mouse APC-IL10, APC-IFNγ, APC-CD25, APC-CD44, APC-CD11c, PerCp-eFlour710-CD4, Alexa488-IL17, PacificBlue-MHC-II I-Ab, PerCpCy5.5-CD62L, PECF594-CD11b, IL4-PE, PE-CD4, PE-Foxp3, PE-CD80, PE-CD86, eflour450-CD8, eFlour450-CD25 and TNFα-eflour450 were purchased from eBioscience (San Diego, CA, USA). Antibodies against mouse FcγII/III receptors (2.4G2, Fc Block) was purchased from BioXcell (West Lebanon, NH, USA).

Peptides

The OVA323-339 peptide (ISQAVHAAHAEINEAGR (SEQ ID NO: 17)) was synthesized by Sigma-Aldrich (Rehovot, Israel). The TYRP1455-463 peptide (AAPDNLGYM, E120), TYRP1111-128 peptide (GTCRPGWRGAACNQKILT, E122), Tyr360-368 peptide (SSMHNALHI, E124) and Tyr99-117 peptide (NCGNCKFGFGGPNCTEKRV, E130) were synthesized at DKFZ (Heidelberg, Germany).

Cloning plasmids and expression vectors

pGEM-4Z 5'UT-eGFP-3'UT-A64 (pGEM-4Z) vector was kindly provided by Dr Eli Gilboa department of Microbiology and Immunology, University of Miami Health system, USA. This plasmid contains a 741-bp eGFP fragment from peGFP-Ni (Clontech), flanked by the 5' and 3' UTRs of *Xenopus laevis* β-globin and 64 A-T bp. The pGEM-4Z-hβ2mKb, pGEM-4Z-hβ2mTLR4 and pGEM-4Z-CLIP vectors were generated previously by the inventors and were used as backbone vectors. mRNA Transcription is controlled by a bacteriophage T7 promoter.

| Insert Name | 5'PCR Forward Primer | 3'PCR Reverse Primer | Product Vector | Designation |
|---|---|---|---|---|
| TYRP $1_{(455-463)}$ variant 2 (E120) | TGTCTCACTGACCGGCTTGTATGCTGCCGCCCCCGACAACCTGGGCTACATGGGAGGTGGCGGATCCGGAGGTGGTT (SEQ ID NO: 18) | AACCACCTCCGGATCCGCCACCTCCCATGTAGCCCAGGTTGTCGGGGGCGGCAGCATACAAGCCGGTCAGTGAGACA (SEQ ID NO: 24) | pGEM-4Z-hb2mKb-TRP $1_{455-463}$ | E120-$K^b$ |
| | TGTCTCACTGACCGGCTTGTATGCTGCCGCCCCCGACAACCTGGGCTACATGGGAGGTGGCGGATCCGGAGGTGGTT (SEQ ID NO: 19) | AACCACCTCCGGATCCGCCACCTCCCATGTAGCCCAGGTTGTCGGGGGCGGCAGCATACAAGCCGGTCAGTGAGACA (SEQ ID NO: 25) | pGEM-4Z-hb2mTLR4-TRP $1_{455-463}$ | E120-TLR4 |
| TYRP1($_{111-128}$) (E122) | ACTGGAGAGCCTTCGCATGAAGCTTGGCACCTGCAGGCCCGGCTGGAGGGGCGCCGCCTGCAACCAGAAGATCCTGACCATGGATAACATGCTCCTTGGGCCTG (SEQ ID NO: 20) | CAGGCCCAAGGAGCATGTTATCCATGGTCAGGATCTTCTGGTTGCAGGCGGCGCCCCTCCAGCCGGGCCTGCAGGTGCCAAGCTTCATGCGAAGGCTCTCCAGT (SEQ ID NO: 26) | pGEM-4Z-CLIP-TRP $1_{111-128}$ | E122-CLIP |
| $Tyr_{(360-368)}$ (E124) | TGTCTCACTGACCGGCTTGTATGCTAGCAGCATGCACAACGCCCTGCACATCGGAGGTGGCGGATCCGGAGGTGGTT (SEQ ID NO: 21) | AACCACCTCCGGATCCGCCACCTCCGATGTGCAGGGCGTTGTGCATGCTGCTAGCATACAAGCCGGTCAGTGAGACA (SEQ ID NO: 27) | pGEM-4Z-hb2mKb-$Tyr_{360-368}$ | E124-$K^b$ |
| | TGTCTCACTGACCGGCTTGTATGCTAGCAGCATGCACAACGCCCTGCACATCGGAGGTGGCGGATCCGGAGGTGGTT (SEQ ID NO: 22) | AACCACCTCCGGATCCGCCACCTCCGATGTGCAGGGCGTTGTGCATGCTGCTAGCATACAAGCCGGTCAGTGAGACA (SEQ ID NO: 28) | pGEM-4Z-hb2mTLR4-$Tyr_{360-368}$ | E124-TLR4 |
| $Tyr_{(99-117)}$ (E130) | ACTGGAGAGCCTTCGCATGAAGCTTAACTGCGGCAACTGCAAGTTCGGCTTCGGCGGCCCCAACTGCACCGAGAAGAGGGTGATGGATAACATGCTCCTTGGGCCTG (SEQ ID NO: 23) | CAGGCCCAAGGAGCATGTTATCCATCACCCTCTTCTCGGTGCAGTTGGGGCCGCCGAAGCCGAACTTGCAGTTGCCGCAGTTAAGCTTCATGCGAAGGCTCTCCAGT (SEQ ID NO: 29) | pGEM-4Z-CLIP-$Tyr_{99-117}$ | E130-CLIP |

Expression vectors were cloned by Restriction Free (RF) method (22). All PCR reactions were done using the Phusion high fidelity PFU DNA polymerase (Thermo scientific). Due to the high length of the mega-primers, all primers were synthesized and PAGE purified by Sigma Aldrich. The sequences of the mega-primers and the resulting vectors are listed in Table1.

Table 1: Mega-primers used in RF cloning process

*In vitro* mRNA transcription

Template DNA cloned in the pGEM4Z-A64 vector was prepared using NucleoBond Xtra Maxi Plus DNA purification system (Macherey- Nagel, Bethlehem, PA, USA) and linearized via the SpeI restriction site positioned at the 3' end of the poly (A) tract of the vector. One □g of linear plasmid was used for in vitro mRNA transcription with T7mScript Standard mRNA Production System (CELLSCRIPT, Madison, U.S.A.) The concentration and quality of the mRNA was assessed by spectrophotometry.

Tetramer preparation and staining

Monomers of biotinylated H-2b mouse MHC-II molecules loaded with either the TYRP1111-128 peptide (GTCRPGWRGAACNQKILT) or Tyr99-117 peptide (NCGNCKFGFGGPNCTEKRV), were obtained from the NIH tetramer facility. Tetramers were freshly prepared by conjugation of the monomers to APC-Strepavidin (ProZyme, Hayward, CA, USA) according to the NIH tetramer protocols. Briefly 130μl of SA-APC (1mg/ml) were added to 100μl of monomer in 10 portions, 10 minutes apart, at room temperature or at 370C. One million of cells in a total volume of 100μl PBS supplemented with 0.5% BSA and 0.1% Na-Azide, were stained with tetramers at 1:100 or 1:200, dilutions for 2hrs. Antibodies to cell surface markers were added for 30min on ice. Cells were then washed, resuspended in PBS supplemented with 0.1% Na-Azide and DAPI and analyzed by SORP LSRII (Becton Dickinson (BD), San Jose, CA, USA).

Generation of DCs from murine bone marrow cells

Murine bone marrow-derived DCs (BMDCs) as described by Lutz et al. (23) were used with minor modifications. Briefly, bone marrow cells from femurs and tibiae of 4-5 weeks old C57Bl/6 female mice were cultured in RPMI medium supplemented with 10% heat-inactivated FCS, 50μM β-mercaptoethanol, 2mM L-glutamine, combined antibiotics and 200U/ml rmGM-CSF (Prospec, Rehovot, Israel). On day 8 non-adherent cells were harvested and further cultured in fresh medium containing 100U/ml rmGM-CSF for 24hrs. DCs were kept immature, or matured by addition of 1μg/ml of lipopolysaccharide (LPS, Sigma, Saint Louis, MI) for another period of 24hrs. Non-adherent cells were analyzed by FACS and showed to express typical characteristics of immature and mature DCs (CD11c+, CD80+, CD86+, MHC-II+).

mRNA electroporation

The procedure was performed as previously described by Cafri et al (17). Briefly, BMDCs cultured for 10 days, were washed twice with OptiMEM medium (GibcoBRL) resuspended in 150μl OptiMEM medium containing 10-20μg transcribed mRNA and electroporated in a 2mm cuvette using BTX ECM 830 electroporator (BTX, Holliston, MA) at 400V, 0.9ms, one pulse. Cells were resuspended in 5mL growth medium and transferred into 50ml tubes for further incubation. Construct's expression was assessed by flow cytometry 6hrs after electroporation. For the MHC-I constructs, cells were stained with anti-human β2m which is highly homologous to mouse b2m and does not interfere with the MHC-I complex.

*In vitro* T cell proliferation assay

Mice were immunized intra-foot pad (i.f.p.) with 50 μg of peptide emulsified at 1:1 ratio in incomplete Freund's adjuvant (IFA). Ten days later the popliteal lymph nodes were excised, washed and re-suspended to 5x106 cells/ml in lymphocyte medium (RPMI medium supplemented with 10% FCS, 2mM glutamine, 1mM sodium pyruvate, 1% nonessential amino acids, 50μM β-mercaptoethanol and combined antibiotics). One hundred μl of cells (5x105 cells) were cultured in 96 flat bottom wells in presence of specific or non-specific peptide concentrations, for 72hrs at 37C, 5% CO2.

For specific proliferation of $CD4^+$ cells derived from OT-II mice, splenocytes purified using IMag beads (BD), according to manufacturer's instructions. Fifty thousand cells were cultured, at several ratios, with DCs that were loaded with peptides or electroporated with constructs. Plates were incubated at 370C, 5% CO2 for 48hrs and then pulsed with 1μCi $^3$H-Thymidine (Perkin Elmer, Boston, MA),) for an additional period of 18hrs. $^3$H-Thymidine uptake was measured using Packard Matrix 96 direct b-counter (Meriden, CT, USA).

CTL in vitro killing assay

Mice were immunized, 3 times at weekly intervals, intraperitoneally (I.P.) with 5x105 mRNA electroporated BMDCs. Ten days after the last vaccination; splenocytes were excised and sensitized with 50μg/ml of peptide for 5 days. The killing protocol was previously described and uses of 35S-methionine as a target cell label in long-term cytotoxic assays (24). Briefly, cells are washed and separated on Lympholyte-M gradient (Cedarlane, Burlington, Canada) for 30min, 2400rpm at 180C. Cells were resuspended in lymphocyte medium and seeded at several concentrations ranging from 5x105- 6x104 in U bottom microplates. Five thousands of 35S methionine (Perkin Elmer) labeled target cells were added to each well. The Plates were incubated for 5hrs at 370C and centrifuged at 1000rpm for 10min at 40C. An aliquot of 50μl was removed to new U bottom 96 well plates followed by incubation with scintillation fluid for at least 4hrs at room temperature. Counts were determined using Packard Matrix 96 direct b-counter. Killing is calculated as follows:

$$\left(\frac{Target_{measured} - Target_{spontanous}}{Target_{total} - Target_{spontanous}}\right) \times 100$$

CTL in vivo killing assay

Mice were immunized, 3 times at weekly intervals, I.P. with 5x105 mRNA electroporated BMDCs. Ten days following the last vaccination, mice were injected intravenously (I.V.) with target cells consisting of splenocytes from B6.SJL (CD45.1+) mice, labeled with CFSE at various concentrations and loaded with specific peptides at 20x106/mice at 1:1:1 ratio. After 18 hours spleens were excised, labeled with anti CD45.1 Ab and analyzed by flow cytometry for specific killing. Killing is calculated as:

$$\left\{1-\left[\left(\frac{\%pop_{high}(day1)}{\%pop_{high}(day0)}\right)\div\left(\frac{\%pop_{medium}(day1)}{\%pop_{medium}(day0)}\right)\right]\right\}\times 100$$

Flow Cytometry

Cell surface staining: Cells were harvested, washed once with cold FACS buffer (PBS+0.5% BSA+0.1% Na-Azide), and incubated for 30min at 40C in the dark with antibodies (at the concentrations recommended by the manufacturer). Cells were washed once using 3ml FACS buffer, resuspended in 0.5ml PBS with 0.1% sodium azide and analyzed by flow cytometry.

Intracellular staining: Cells in lymphocyte buffer were incubated for 2hrs at 370C, 5%CO2 with 50ug/ml peptides or a mixture of 50ng/ml Phorbol Myristate Acetate (PMA) and 750ng/ml Ionomycin (both from Sigma). Then, a mixture of 2μM Brefeldin A (BFA) and 3ug/ml Monensin final concentration (both from eBioscience) was added to the culture, fd-lowed by 4hrs of incubation. Cells were washed and fixed using 4% p-formaldehyde (PFA) for 15min. Permeabilization followed using PBS (w/o Ca and Mg) supplemented with 0.1% saponin, 5% FCS and 0.1% Na-Azide, for 15mm at 40C. FcγII/III Block (1μg/sample, eBioscience) was then added for 15min followed by antibodies (at the concentrations recommended by the manufacturer). Cells were washed once using 3ml Permeabilization buffer, resuspended in 0.5ml PBS with 0.1% Na-Azide and analyzed by flow cytometry. All samples were analyzed by SORP LSRII (Becton Dickinson, San Jose, CA, USA) and FlowJo software (ThreeStar, San Carlos, CA, USA).

Tumor growth experiments

Eight mice per group were inoculated I.F.P with $1x10^5$ Ret cells. Tumor size was monitored daily by calipers. When the tumors reached 8 mm in diameter, mice were sacrificed.

Statistical analysis

Statistical analysis was done using GraphPad Prism7 software. Data are presented as the mean ±SEM. Statistical significance was assessed by the 1way ANOVA or 2way ANOVA according to data requirements. Bonferroni and Tukey's posttests were followed according to their appropriate use. Survival curves were generated using the product limit method of Kaplan and Meier, and comparisons were made using the log-rank test. In all cases, a P value of <0.05 was considered statistically significant. *P<0.05, **P<0.01, ***P<0.001.

**Example 1: Gene assembly**

The inventors have recently described the development and assessment of recombinant bifunctional b2m-based polypeptides, which couple MHC-I presentation to constitutive TLR4 activation. The constructs are comprised of an antigenic peptide linked to the β2-microglobulin and to the cell membrane via an anchor sequence. The anchor is either the Kb, which allows better presentation on the cell surface or the intracellular portion of TLR4, which confers a mature phenotype of BMDCs. Therefore, the inventors immunized with both anchors for the same antigenic peptide, i.e. $K^b$ and TLR4 in 1:1 ratios. The inventors have previously generated chimeric polypeptides with MAAs specific to $gp100_{25\text{-}33}$ and $TYRP2_{180\text{-}188}$ and tested their antitumor activity both in a transplantable B16 and in a spontaneous *ret* melanoma model. To broaden the clinical potential of the constructs and to allow combinations with MHC-I MAAs, the inventors used the SYFPEITHI prediction software. Among several MHC-I tested peptides, two peptides were chosen: $TYRP1_{455\text{-}463}$, which binds to the H-2Db and was previously reported to confer anti-tumor immune responses and $Tyr_{360\text{-}368}$ that was predicted by SYFPEITHI prediction software to bind to the H-2Db. These peptides were assembled into the chimeric platform with both the TLR4 and the $K^b$ anchors. The design of the constructs is described in Figures 1A and 1B.

Since the anti-tumor activity of $CD8^+$ CTLs is enhanced and sustained by $CD4^+$ T helper (Th) cells, the inventors aimed at developing the universal MHC-II platform composed of a chimeric Ii, in which the CLIP semi peptide (that stabilizes the MHC-II molecule), is replaced by an antigenic peptide. The inventors assumed that this will allow a better presentation of the MHC-II-peptide complex on the cell surface and will induce the $CD4^+$ T cell immune response which will assist in eradicating the tumor. Among several MHC-II tested peptides, the $TYRP1_{111-128}$ peptide, previously reported to confer an immune response and the $Tyr_{99-117}$ peptide that was predicted to induce an immune response by the IEDB prediction tool. Both peptides were assembled into the chimeric platform. The design of the constructs is described in Figure 1C and 1D. The designation of new constructs and the immunization groups are shown in Figure 1E.

**Example 2: BMDCs present the MHC-I constructs on their cell surface and induce CTLs**

MHC-I constructs expression was monitored on the cell surface by flow cytometry with anti-human β2m antibody. Expression kinetics of each of the MHC-I constructs with either the $K^b$ or TLR4 anchors for up to 48hrs following mRNA electroporation was similar to previously described. Mice were immunized with mRNA electroporated BMDCs and then tested for presence and activity of CTLs. Figure 2A demonstrates a specific killing of peptide-loaded target cells in an *in vivo* assay. Figure 2B shows the killing of melanoma cell lines following immunization in an *in vitro* assay. Thus, the constructs are presented by the electroporated BMDCs and induce specific CTLs.

**Example 3: BMDCs present the MHC-II constructs on their cell surface**

The CLIP constructs do not interfere with the endogenous MHC-II molecule assembly or modify it. Since antibodies against the specific complex of MHC-II and the peptide are not commercially available, the most efficient way to validate presentation is by a functional proliferation assay. The inventors demonstrated that electroporated BMDCs with the OVA-CLIP construct, either immature or mature, induced specific proliferation of OT-II $CD4^+$ T cells in a similar manner to peptide-loaded BMDCs (Fig. 3A). BMDCs electroporated with CLIP-E122 (TYRPi-MHC-II) and CLIP-E130 (Tyr-MHC-II) constructs were able to re-stimulate and induce proliferation of $CD4^+$ T cells following immunization with TYRP1 or Tyr peptide respectively (Fig. 3B, C). To further verify that proliferating cells display a peptide-specific TCR the inventors stained for MHC-II tetramers and demonstrated that peptide specific T cells expanded following the immunization protocol with BMDC electroporated with class II constructs (Fig. 3D).

Immunization with MHC-I constructs inhibits tumor growth and improves mouse survival Here, on day 0, mice were inoculated with Ret melanoma cells and monitored daily (Fig. 4A). The inventors found that immunization with a combination of E120-TYRP1 and E124-Tyr MHC-I constructs significantly inhibited tumor growth, reduced the average tumor size and prolonged mouse survival as compared to all other groups (Fig. 4B-D). Vaccination with E120-TYRP1 inhibited tumor growth to a lesser extent, whereas E124-Tyr alone did not affect at all.

**Example 4: Co-immunization with MHC-II CLIP constructs inhibits tumor growth and results in increased survival**

Next, BMDCs were simultaneously electroporated with MHC-I and MHC-II constructs. There are multiple combinations applicable for immunization. In this study, the inventors focused on MHC-I and MHC-II antigens derived from the same protein. It was shown that for the TYRP1 peptides, the combination of MHC-I and MHC-II constructs is highly beneficial in reducing the average tumor size, prolonging survival and even eradicating tumors in some mice (Fig. 4E-G). Three mice that did not develop tumors following vaccination were rechallenged with the Ret tumor cells on day 78 (opposite foot). Two mice, in this group, completely rejected the tumor and in the third mouse, the tumor grew slowly than in the control. Furthermore, the inventors found that for Tyr peptides, the immunization with the MHC-I and MHC-II combination resulted in reduced tumor size, prolonged survival and complete prevention of tumors growth in 5 out of 8 mice (Fig. 4H-J). On the other hand, administration of a single CLIP construct alone, for both TYRP1 and Tyr, did not affect tumor development.

**Example 5: Immunization with the constructs generates CTLs, Th1 and Th2 immune responses**

The inventors aimed at investigating the mechanism, which enhances the immune response following vaccination with the constructs. Analysis of splenocytes derived from tumor bearing mice 10 days after the last immunization revealed several changes in lymphocyte populations (Fig. 5). Significantly higher frequencies of $CD8^+IFN\gamma^+$ cells were found in all mice immunized with the E120-TYRP1 MHC-I construct as compared to the control group. The same trend for $CD8^+IFN\gamma^+$, $CD8^+TNF\alpha^+$ and $CD8^+IFN\gamma^+TNF\alpha^+$ T cells was observed in groups immunized with E120-TYRP1 and E124-Tyr constructs, although this increase was not statistically significant. Groups immunized with a single MHC-II construct do not show elevated levels of these T cells.

Next, the inventors demonstrated that the frequency of $CD4^+IFN\gamma^+$, $CD4^+TNF\alpha^+$ and $CD4+IFN\gamma+TNF\alpha+$ is higher in groups immunized with both of the MHC-I constructs (E120-TYRP1 and E124-Tyr) and in the group immunized with the MHC-I and MHC-II TYRP1 combination (E120+E122-CLIP) as compared to the control group. The same changes were observed for the MHC-I and MHC-II Tyr combination (E124+E130-CLIP) (Fig. 6).

In order to validate functional CTLs, the inventors performed an *in vivo* killing assay of tumor bearing mice 10 days following last immunization. Substantial killing of peptide loaded target cells was observed in all the groups that were immunized with an MHC-I construct (E120-TYRP1 or E124-Tyr) (Fig 7A). These CTLs are highly specific and more potent following immunization with the E120-TYRP1 construct, consistent with the killing observed in naive mice (Fig. 2B). Furthermore, the administration of the CLIP construct resulted in increased frequencies of $CD4^+IL4^+$ T cells in the MHC-I and MHC-II TYRP1 combination (E120+E122-CLIP) and the same trend is observed for the MHC-I and MHC-II Tyr combination (E124+E130-CLIP) (Fig. 7B).

In addition, the inventors could not find any significant differences in other immune populations such as $CD4^+Foxp3^+IL10^+$ regulatory T cells (Tregs) and $CD11b^+Gr1^+$ myeloid-derived suppressor cells (MDSCs). Regarding $CD4^+IL17^+$ T cells the inventors observed a significant elevation of these cells only in the MHC-I and MHC-II Tyr combination (Fig.8).

**Discussion**

Following the design and generation of inventive constructs with melanoma associated antigens (MAAs) for both MHC-I and MHC-II, they were electroporated into BMDCs and were expressed on the cell surface. MHC-I constructs induced specific $CD8^+$ CTLs immune response, whereas MHC-II constructs induce proliferation of specific $CD4^+$ cells. The therapeutic potential of the constructs was tested in mice transplanted with Ret melanoma cells. This cell line originated from the skin tumors of *ret* transgenic mice expresses such MAAs as gp100, TYRP1, TYRP2 and Tyr. The inventors showed that the immunization with the constructs of the invention had a remarkable therapeutic potential. In groups immunized with a combination of MHC-I constructs, i.e. BMDCs electroporated with both TYRP1 and the Tyr antigens, the inventors demonstrated a significant inhibition of tumor growth, a reduction in averaged tumor size and a prolonged survival. Such results might indicate that multiple antigenic presentations are required to exert an efficient anti-tumor immune response.

Moreover, a significant inhibition of tumor growth, reduction in average tumor size and prolonged mouse survival was observed following vaccination with the combination of MHC-I and MHC-II constructs, either of TYRP1 or Tyr antigens. Such combination elicited a sufficient and more sustained anti-tumor immune response. The CLIP chimeric constructs administered alone have no effect on the tumor growth. However, in combination with the MHC-I construct, it has a tremendous impact on attenuating the tumor development to almost complete rejection. This was observed for both the TYRP1 and the Tyr antigens, indicating on the generation of a strong immune response. This was strengthened by the fact that two out of three rechallenged mice from the TYRP1 E120+E122-CLIP group rejected the tumor completely and the third mouse exhibited slower tumor growth kinetics compared to control.

Analyzing the mechanism underlying the exerted immune response, the inventors detected increased frequencies of $CD8^+IFN\gamma^+$ and $CD4^+IFN\gamma^+$, $CD4^+TNF\alpha^+$ and $CD4^+IFN\gamma^+TNF\alpha^+$ suggesting a Th1 immune response and efficient killing capacity of the tumor. In all groups immunized with BMDCs electroporated with MHC-I construct, either in combination or alone, CTLs were able to eliminate loaded target cells. Higher levels of $CD4^+IL4^+$ (indicating a Th2 immune response) were detected in groups immunized with BMDCs electroporated with MHC-II constructs, either alone or in combination with MHC-I construct. This profiling trend was seen for both of TYRP1 and Tyr, although not always in a statistically significant manner, likely due to the small numbers of mice per group (n=3).

Tregs play a major role in maintenance of self-tolerance in healthy hosts, and may hamper an effective antitumor immune response. Importantly, the inventors found a non-significant trend of elevated frequencies of these immunosuppressive cells in control groups compared to construct immunized groups. Another crucial immunosuppressive cell population is represented by MDSCs that inhibit antitumor T-cell responses by multiple mechanisms and can be considered as one of the most important components of tumor-induced immunosuppression in melanoma. The inventors observed also a trend of higher frequencies of Th17 cells among the construct-immunized groups as compared to the control. Th17 cells have been shown to be potent inducers of tissue inflammation and have been associated with inflammatory conditions. Moreover, it was recently identified that Th17 cells are protective for melanoma metastases in a mouse model.

To summarize, the inventors provide a novel mRNA vaccine platform, which is highly efficient in eradicating the tumor. The system is modular and can be applied on multiple cancers. The presentation of both MHC-I and MHC-II restricted MAAs on BMDCs has a potent anti-tumor effect, mediated by a systemic and comprehensive immune response. The wide range of MHC-I and MHC-II constructs developed by the inventors enables investigation of other potential combinations to increase further the efficiency of tumor immunotherapy.

## Claims

1. An method for the production of a cellular tumor vaccination composition, the method comprising the steps of:

   (a) Providing antigen presenting cells (APCs),
   (b) Introducing into said APCs one or more genetic expression constructs selected from:

      (i) A Major Histocompatibility Complex (MHC) class I fusion protein comprising an N-terminal MHC class I antigenic peptide, a β2 microglobulin (β2 m), and a C-terminal membrane anchor, and
      (ii) A chimeric invariant chain (Ii), wherein the CLIP sequence is replaced by a sequence comprising an MHC class II antigenic peptide;

   (c) Expressing the selected genetic expression constructs of (b) in said APCs, and
   (d) Harvesting said APCs of (c) to obtain the cellular tumor vaccination composition.

2. The method according to claim 1, wherein the antigen presenting cell is a bone marrow dendritic cell (BMDC), B cell, dendritic cell, macrophage, activated epithelial cell, fibroblast, thymic epithelial cell, thyroid epithelial cell, glial cell, pancreatic beta cell, and a vascular endothelial cell.

3. The method according to any one of claims 1 to 2, wherein the genetic expression construct is an mRNA expression vector, and wherein the introduction of said genetic expression constructs into said antigen presenting cells is performed by RNA electroporation.

4. The method according to any one of claims 1 to 3, wherein the C-terminal membrane anchor is selected from $K^b$ or a partial Toll like receptor (TLR)4 or a partial TLR2 protein comprising a transmembrane domain and/or an intracellular signaling portion, preferably a TLR4 intracellular signaling portion.

5. The method according to any one of claim 1 to 4, wherein step (b) comprises introducing into said APCs genetic expression constructs according to (i) and (ii).

6. The method according to any one of claims 1 to 5, wherein the MHC class I antigenic peptide and the MHC class II antigenic peptide are selected from

   (a) antigenic peptide sequences of at least two different tumor associated antigens (TAA), and wherein the two different TAA are associated with the same tumor, such as melanoma; or
   (b) antigenic peptide sequences of the same TAA.

7. The method according to claim 6, wherein the TAA selected from gp100, tyrosinase (Tyr), tyrpsinase related protein (TYRP)1 or TYRP2.

8. The method according to any one of claims 1 to 7, wherein between steps (c) and step (d) the APCs are cultured and/or expanded.

9. A cellular composition obtainable by a method according to any one of claims 1 to 8.

10. A cell, comprising a genetic expression construct system, comprising one or more genetic expression constructs, or expression products of at least one genetic expression constructs according to step (b) (i) and/or (ii) of any one of claims 1 to 8.

11. A nucleic acid comprising a nucleotide sequence of a genetic expression construct of step (b) (ii) according to any one of claims 1 to 8.

12. An antigenic peptide, comprising a sequence according to any of SEQ ID NO: 3 to 14, or a variant antigenic peptide, comprising a sequence according to any of SEQ ID NO: 3 to 14 with not more than 3 amino acid substitutions, deletions, additions or insertions compared to these sequences in each case independently.

**13.** A chimeric CD74 protein, comprising a sequence wherein the MHC class II-associated invariant chain (Ii)-derived peptide (CLIP) is replaced by a sequence selected from any one of SEQ ID NO: 3 to 6.

**14.** A nucleic acid comprising a nucleotide sequence encoding for the antigenic peptide according to claim 12, or the chimeric CD74 protein according to claim 13, preferably wherein the nucleic acid is an expression vector.

**15.** A recombinant host cell comprising the antigenic peptide according to claim 12, or the chimeric CD74 protein according to claim 13, or the nucleic acid according to claim 14.

**16.** A product for use in medicine, preferably for the treatment of a tumor disease, wherein the product is a cellular composition according to claim 9, a cell according to claim 10, a nucleic acid according to claim 12, the antigenic peptide according to claim 12, the chimeric CD74 protein according claim 13, the nucleic acid according to claim 13, or the recombinant host cell according to claim 15.

Figure 1:

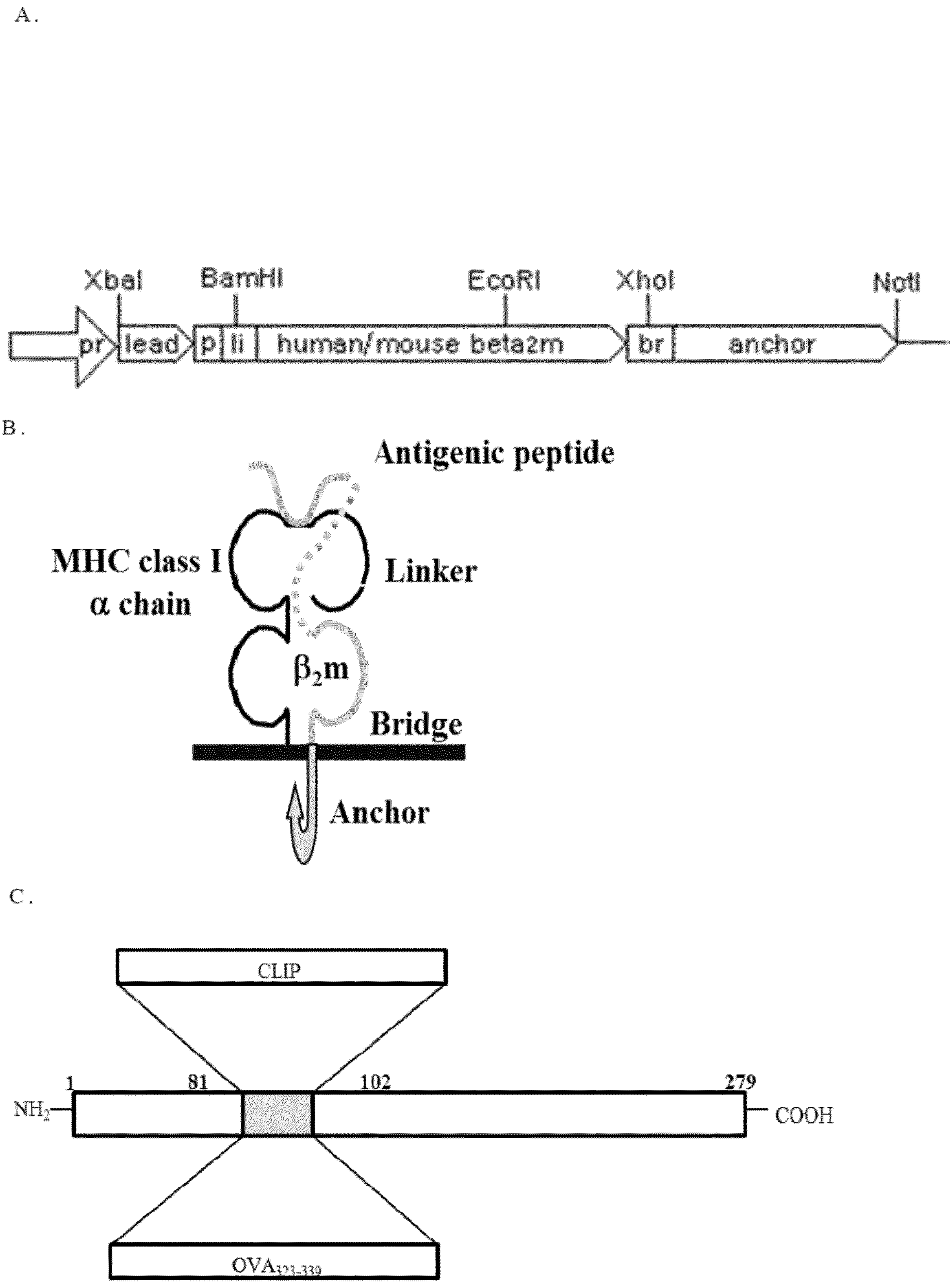
A.
XbaI
BamHI
EcoRI
XhoI
NotI
pr
lead
p
li
human/mouse beta2m
br
anchor
B.
Antigenic peptide
MHC class I
α chain
Linker
β2m
Bridge
Anchor
C.
CLIP
1
81
102
279
NH2
COOH
OVA323-339

Figure 1 cont.:

D.

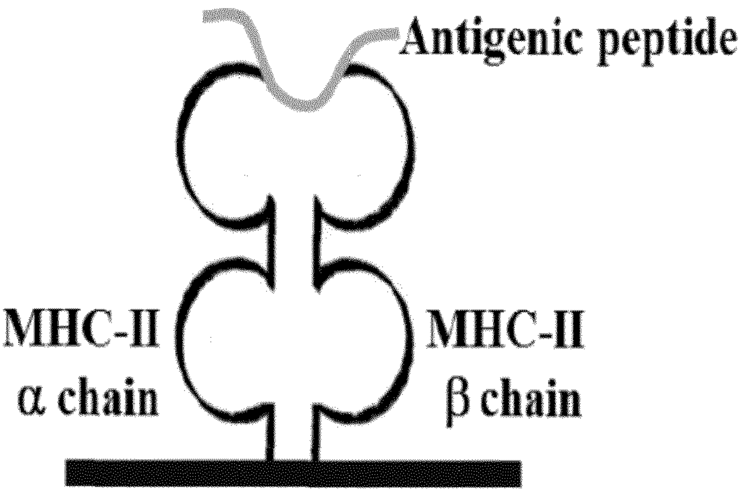


E.

| | Vaccine Construct's Combination | Designation |
|---|---|---|
| MHC-I | $TRP1_{455-463}$-hβ2m-$K^b$ and $TRP1_{455-463}$-hβ2m-TLR4 | E120 |
| | $Tyr_{360-368}$-hβ2m-$K^b$ and $Tyr_{360-368}$-hβ2m-TLR4 | E124 |
| MHC-II | $TRP1_{111-128}$-Invariant Chain | E122-CLIP |
| | $Tyr_{99-117}$-Invariant Chain | E130-CLIP |
| | $OVA_{323-339}$-Invariant Chain | OVA-CLIP |

Figure 2:

A.

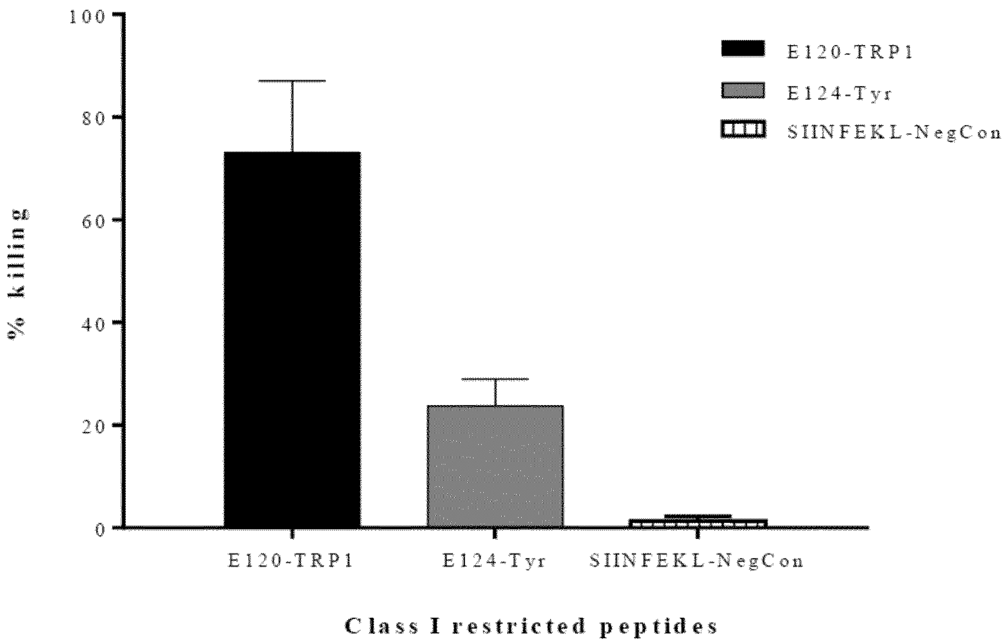
% killing
100
80
60
40
20
0
E120-TRP1
E124-Tyr
SIINFEKL-NegCon
Class I restricted peptides

B. (i)

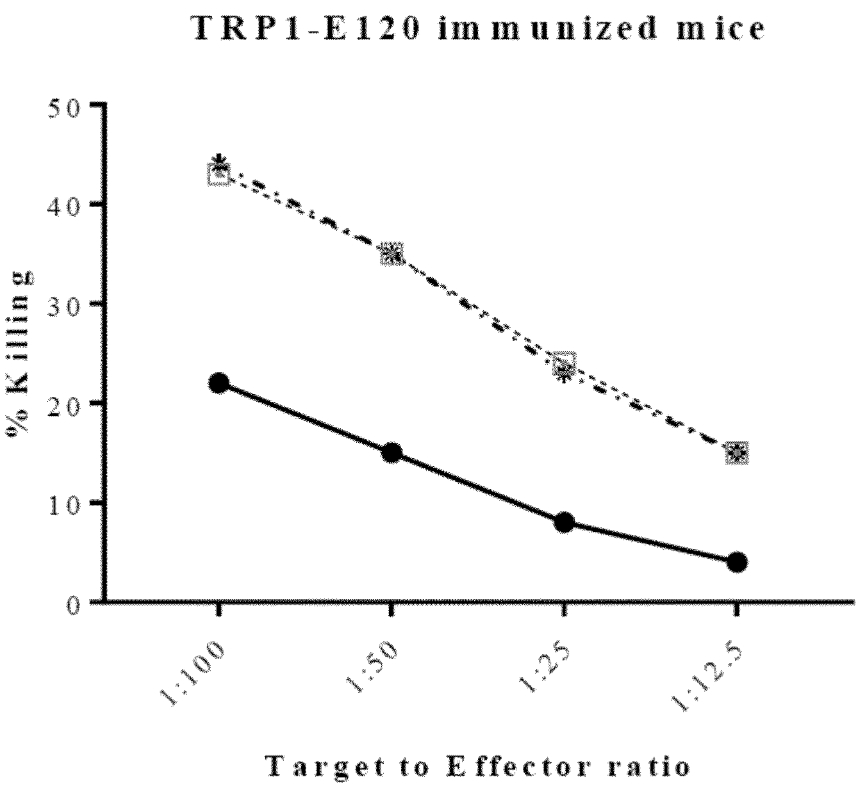
TRP1-E120 immunized mice
% Killing
50
40
30
20
10
0
1:100
1:50
1:25
1:12.5
Target to Effector ratio

Figure 2 cont.:

(ii)

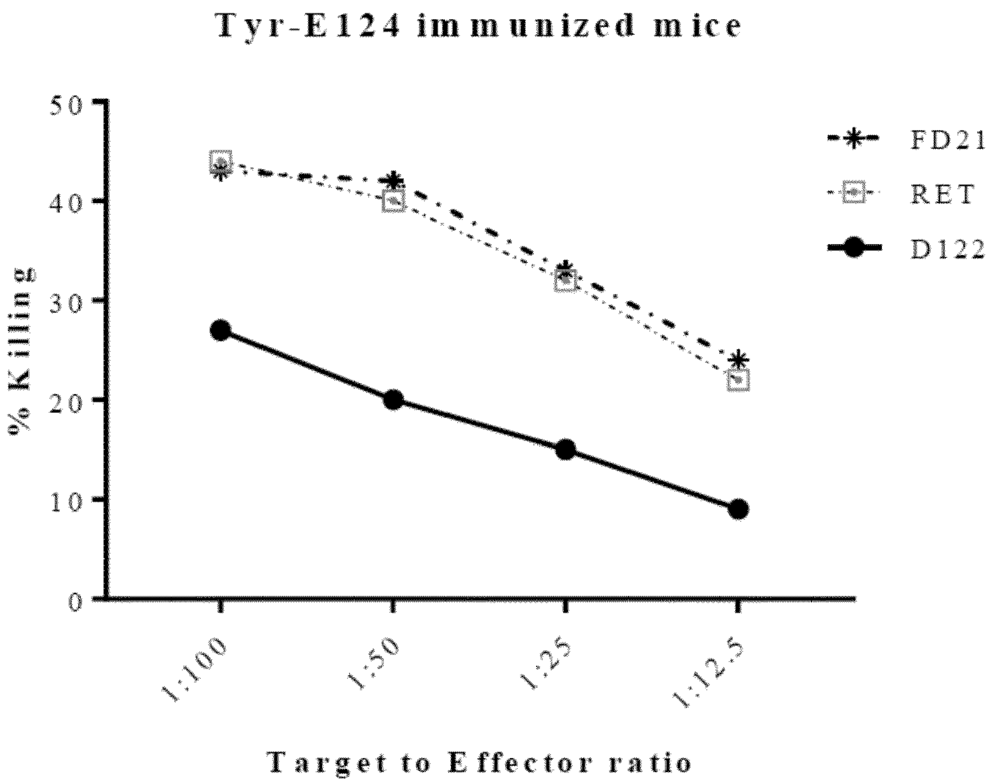

Figure 3:

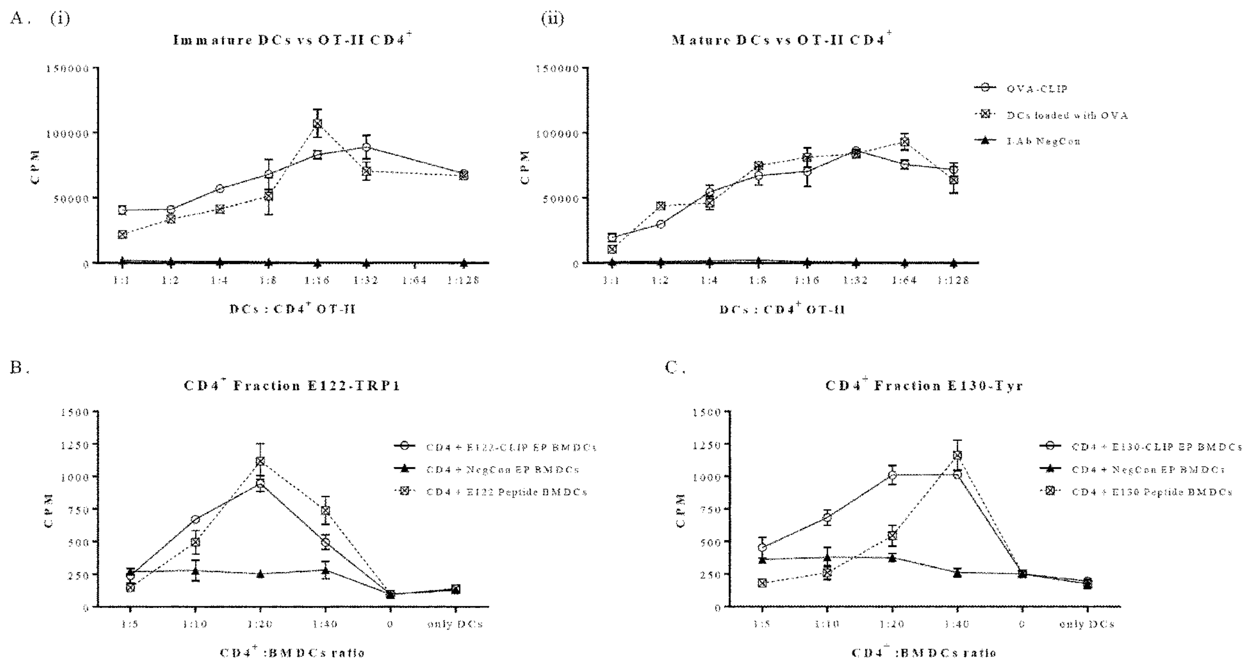
A. (i)
Immature DCs vs OT-II CD4+
CPM
150000
100000
50000
0
1:1 1:2 1:4 1:8 1:16 1:32 1:64 1:128
DCs : CD4+ OT-II
(ii)
Mature DCs vs OT-II CD4+
OVA-CLIP
DCs loaded with OVA
I-Ab NegCon
B.
CD4+ Fraction E122-TRP1
CPM
1500
1250
1000
750
500
250
0
1:5 1:10 1:20 1:40 0 only DCs
CD4+ :BMDCs ratio
CD4 + E122-CLIP EP BMDCs
CD4 + NegCon EP BMDCs
CD4 + E122 Peptide BMDCs
C.
CD4+ Fraction E130-Tyr
CD4 + E130-CLIP EP BMDCs
CD4 + NegCon EP BMDCs
CD4 + E130 Peptide BMDCs

Figure 3 cont.:

D. (i)

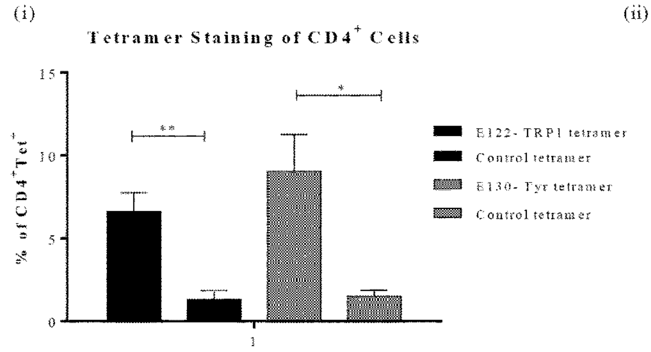


(ii)

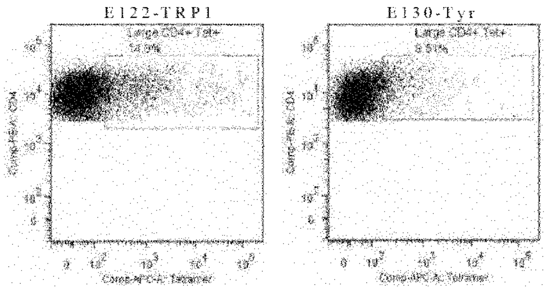

Figure 4:

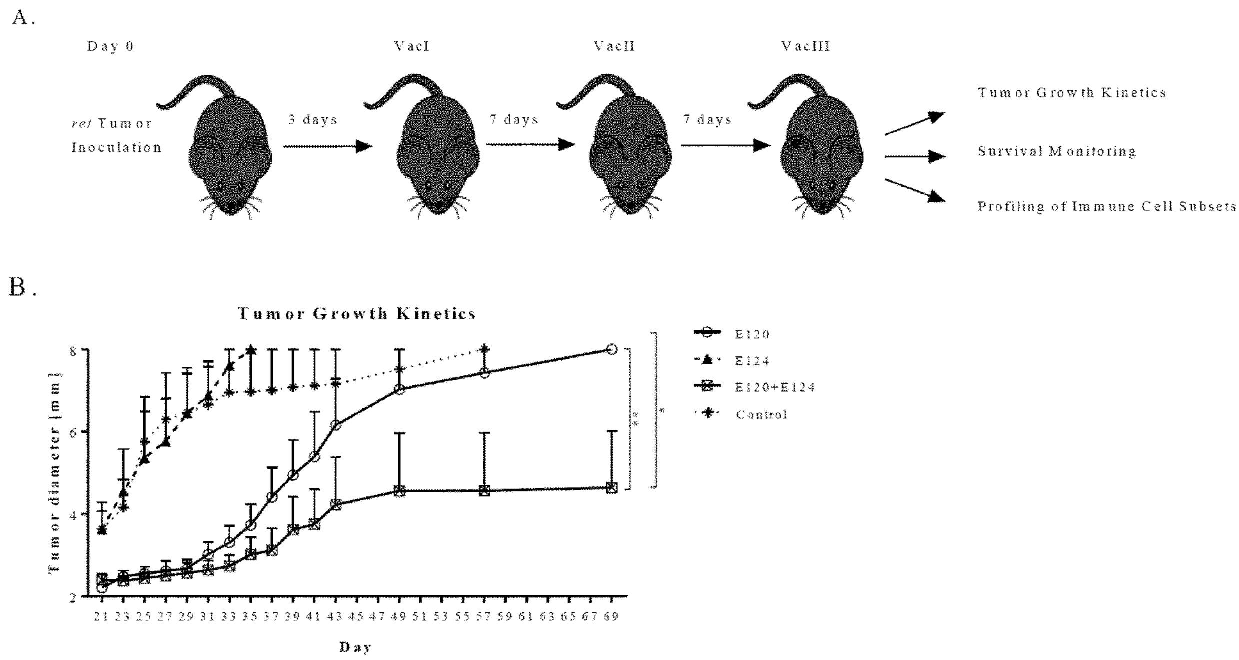
A.
Day 0
ret Tumor
Inoculation
3 days
VacI
7 days
VacII
7 days
VacIII
Tumor Growth Kinetics
Survival Monitoring
Profiling of Immune Cell Subsets
B.
Tumor Growth Kinetics
Tumor diameter [mm]
Day
E120
E124
E120+E124
Control

Figure 4 cont.:

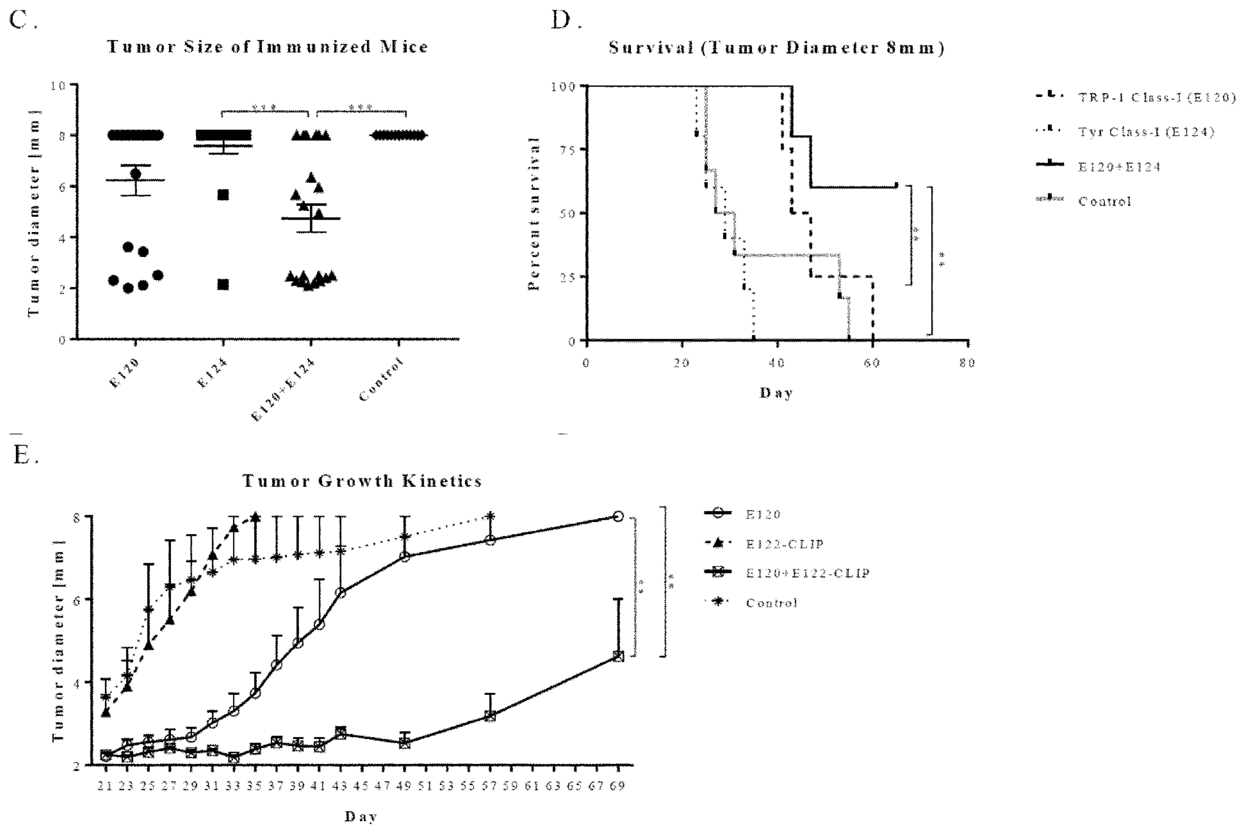

Figure 4 cont.:

F.

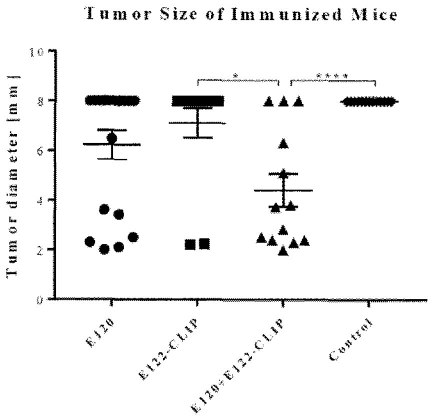


G.

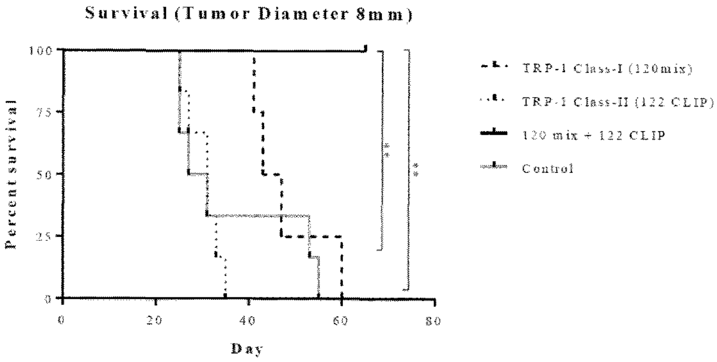


H.

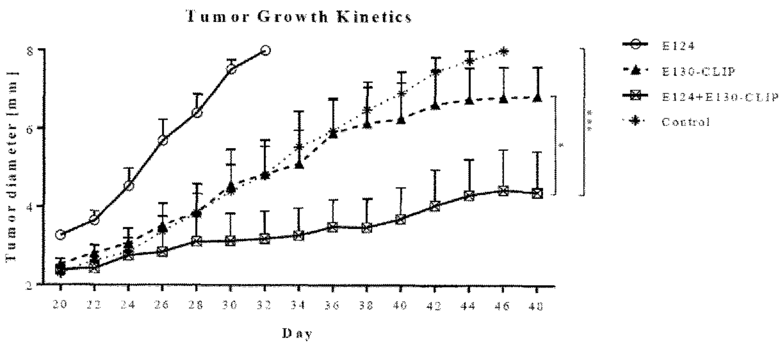

Figure 4 cont.:

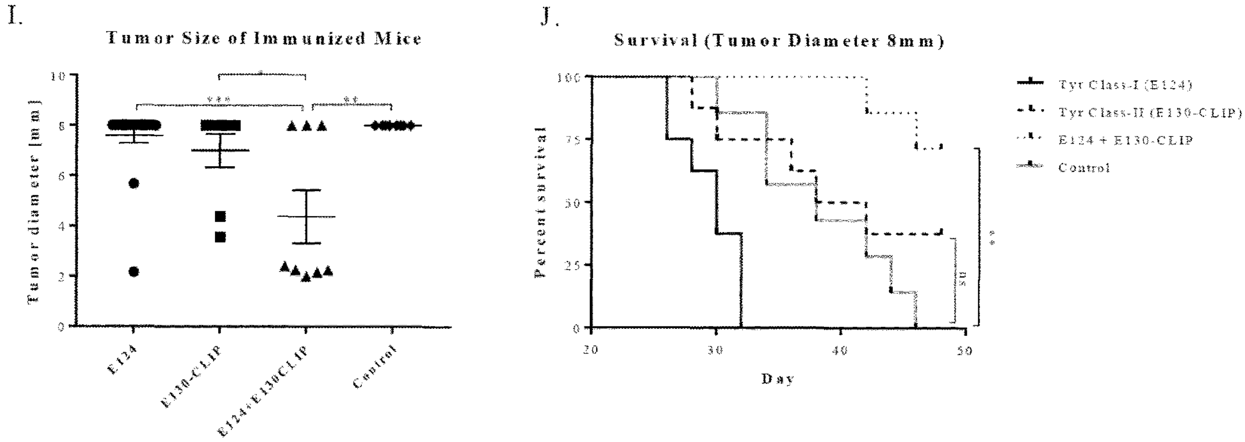

Figure 5:

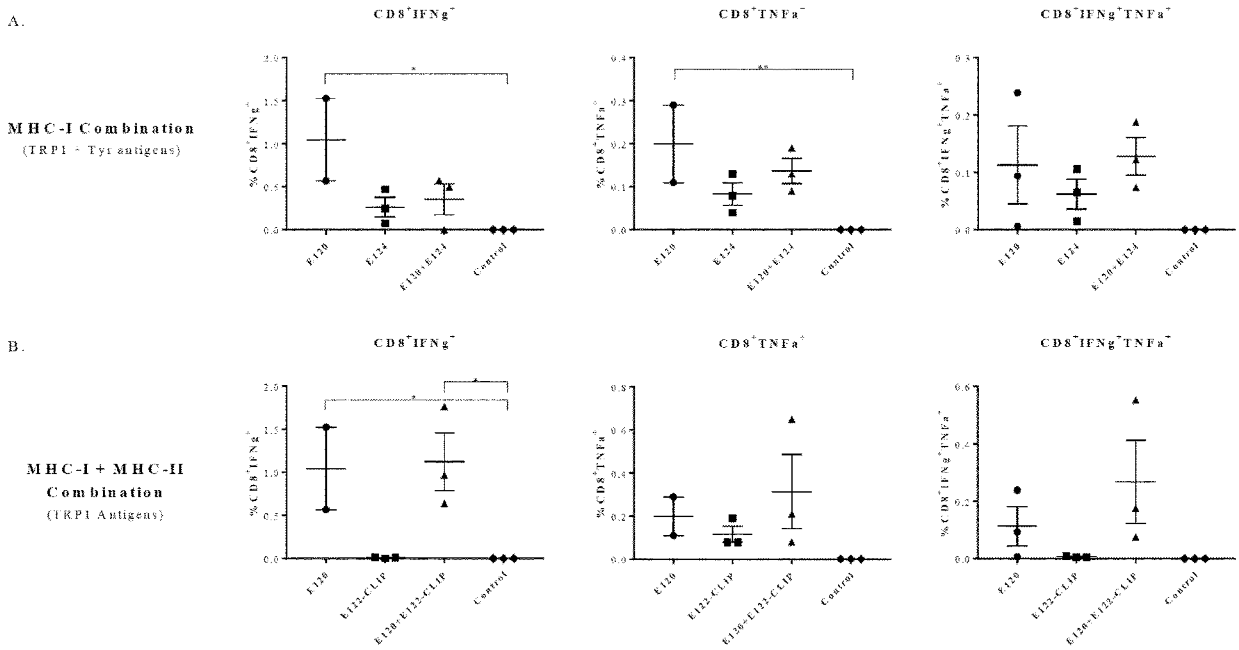
A.
MHC-I Combination
(TRP1 + Tyr antigens)
CD8+IFNg+
CD8+TNFa+
CD8+IFNg+TNFa+
E120
E124
E120+E124
Control
B.
MHC-I + MHC-II
Combination
(TRP1 Antigens)
E122-CLIP
E120+E122-CLIP

Figure 5 cont.:

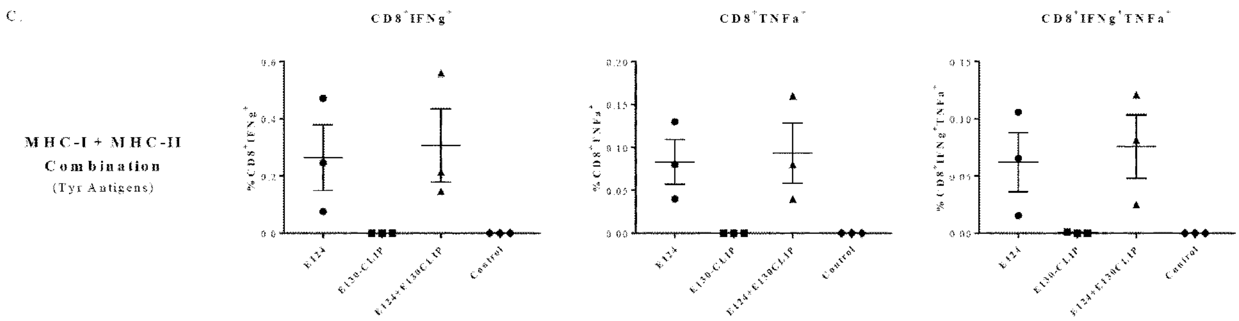

Figure 6:

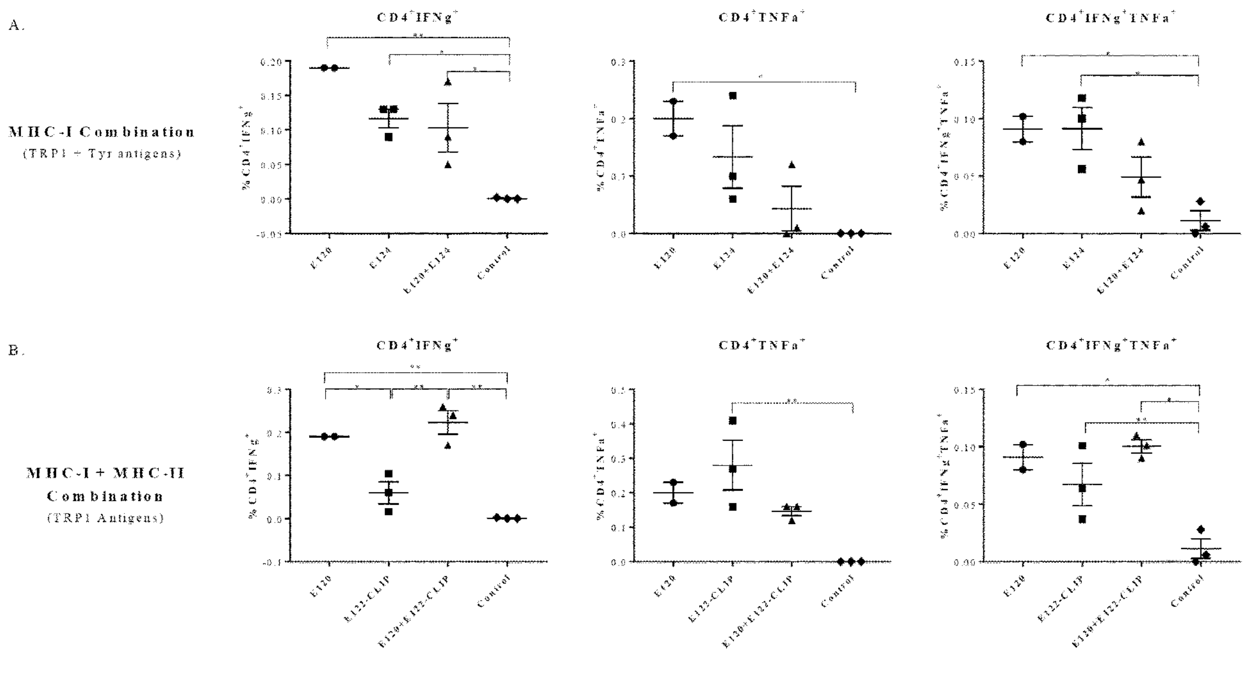
A.
MHC-I Combination
(TRP1 + Tyr antigens)
B.
MHC-I + MHC-II
Combination
(TRP1 Antigens)
CD4⁺IFNg⁺
CD4⁺TNFa⁺
CD4⁺IFNg⁺TNFa⁺
E120
E124
E120+E124
E122-CLIP
E120+E122-CLIP
Control

Figure 6 cont.:

C.

**MHC-I + MHC-II**
**Combination**
(Tyr Antigens)

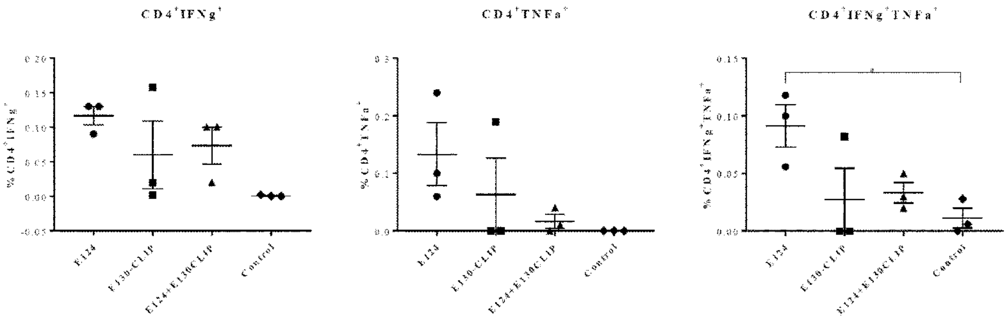

Figure 7:

A.

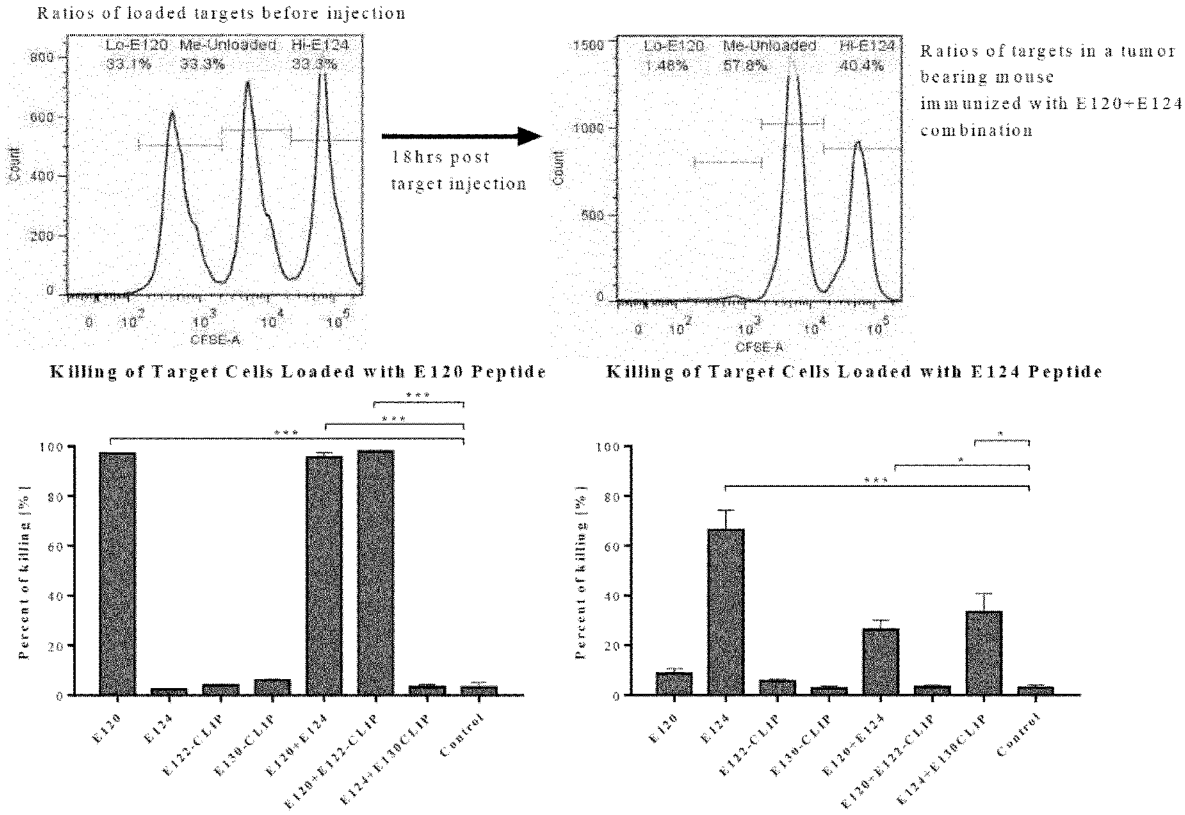
Ratios of loaded targets before injection
Lo-E120 33.1%
Me-Unloaded 33.3%
Hi-E124 33.3%
Count
CFSE-A
18hrs post target injection
Lo-E120 1.48%
Me-Unloaded 57.8%
Hi-E124 40.4%
Ratios of targets in a tumor bearing mouse immunized with E120+E124 combination
Killing of Target Cells Loaded with E120 Peptide
Killing of Target Cells Loaded with E124 Peptide
Percent of killing [%]
E120
E124
E122-CLIP
E130-CLIP
E120+E124
E120+E122-CLIP
E124+E130CLIP
Control

Figure 7 cont.:

B.

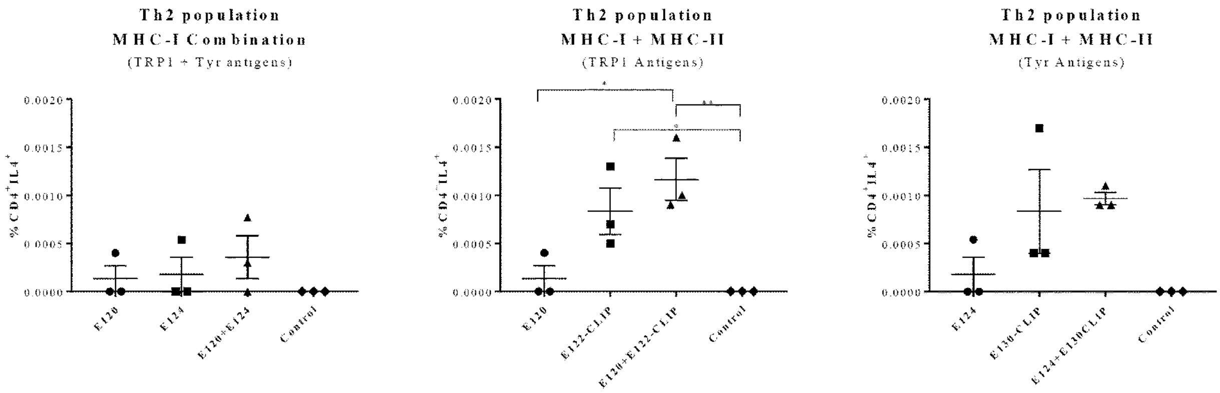

Figure 8:

A.

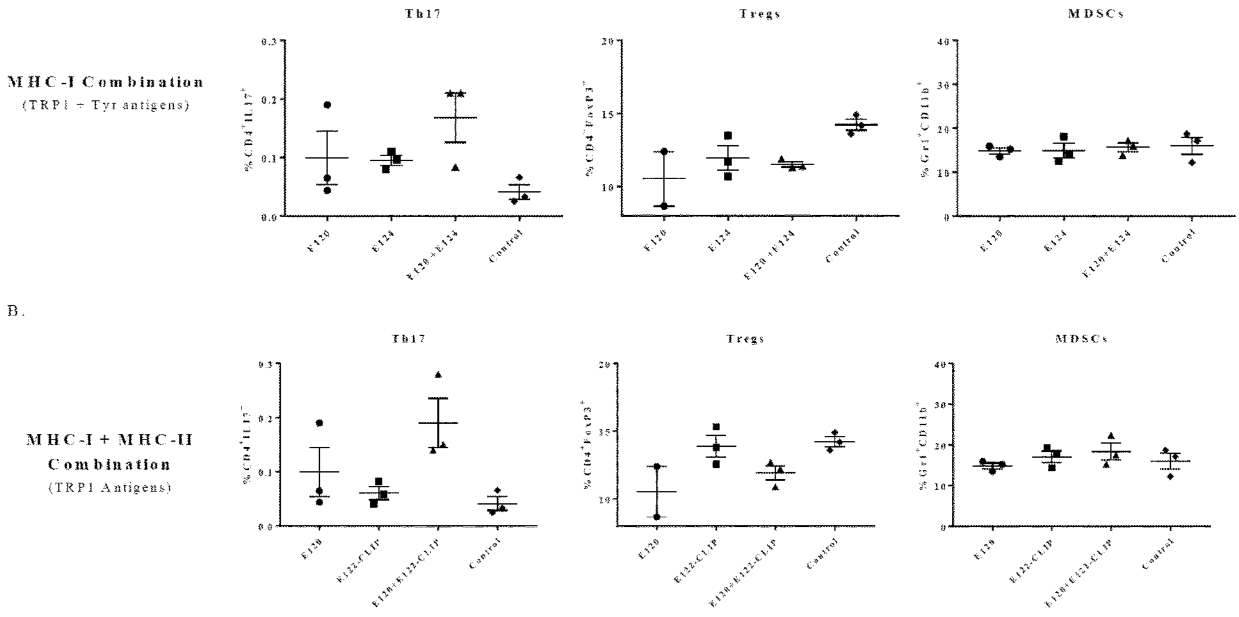
MHC-I Combination
(TRP1 + Tyr antigens)
Th17
Tregs
MDSCs
% CD4+IL17+
% CD4+FoxP3+
% Gr1+CD11b+
E120
E124
E120+E124
Control
B.
MHC-I + MHC-II Combination
(TRP1 Antigens)
E122-CLIP
E120+E122-CLIP

Figure 8 cont.:

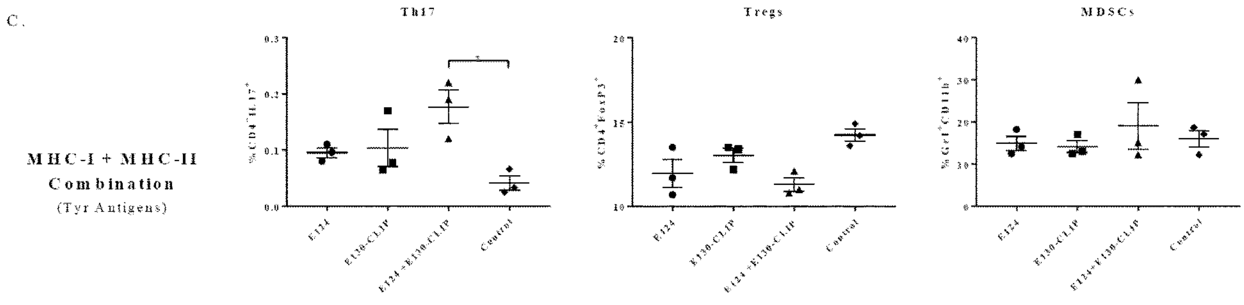

Europäisches Patentamt
European Patent Office
Office européen des brevets

# EUROPEAN SEARCH REPORT

Application Number
EP 18 16 4740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHARBI-YUNGER ADI ET AL: "mRNA-based dendritic cell immunization improves survival in ret transgenic mouse melanoma model.", ONCOIMMUNOLOGY JUN 2016, vol. 5, no. 6, June 2016 (2016-06), page e1160183, XP002785087, ISSN: 2162-4011 * the whole document * ----- | 1-4, 6-10,16 | INV. A61K39/00 C07K14/74 A61P35/00 |
| X | WO 2008/041231 A2 (GAVISH GALILEE BIO APPL LTD [IL]; GROSS GIDEON [IL]; MARGALIT ALON [IL] 10 April 2008 (2008-04-10) * abstract * * page 11 - page 12, line 21 * * page 20, last paragraph * * page 22, line 17 - page 23, line 16 * * page 23, last paragraph - page 24, paragraph 1 * * (xi); page 25 * * (xxiii), (xxiv) and (xxvi); page 26 * * page 29, paragraph 2 * * page 32, paragraph 2 * * page 32, last paragraph - page 33, paragraph 1 * * page 36, paragraph 1 * * page 37, paragraph 1 * ----- -/-- | 1-4, 6-10,16 | |

TECHNICAL FIELDS SEARCHED (IPC)
A61K
C07K
A61P

~~The present search report has been drawn up for all claims~~

4

| Place of search | Date of completion of the search | Examiner |
|---|---|---|
| The Hague | 10 December 2018 | Noë, Veerle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 16 4740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | A. MARGALIT ET AL: "Induction of Antitumor Immunity by CTL Epitopes Genetically Linked to Membrane-Anchored ?2-Microglobulin", THE JOURNAL OF IMMUNOLOGY, vol. 176, no. 1, 19 December 2005 (2005-12-19), pages 217-224, XP055394142, US ISSN: 0022-1767, DOI: 10.4049/jimmunol.176.1.217 * the whole document * ----- | 1,2,4, 6-10,16 | |
| X | US 2007/055049 A1 (GREY HOWARD M [US] ET AL) 8 March 2007 (2007-03-08) * claims 1-2; sequences 3149,2849 * * paragraphs [0089], [0091] - [0092], [0115], [0127], [0346], [0347], [0422] * ----- | 12,14-16 | |
| X | WO 2004/031211 A2 (EPIMMUNE INC [US]; SIDNEY JOHN [US]; SOUTHWOOD SCOTT [US]; SETTE ALESS) 15 April 2004 (2004-04-15) * sequence 2472 * * claims 1,16,17 * * page 3, last paragraph - page 4, paragraph 1 * * page 26, paragraph 3 * * page 32, paragraph 2 * ----- -/-- | 12,14-16 | TECHNICAL FIELDS SEARCHED (IPC) |

4

~~The present search report has been drawn up for all claims~~

| Place of search | Date of completion of the search | Examiner |
|---|---|---|
| The Hague | 10 December 2018 | Noë, Veerle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 16 4740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/45954 A1 (EPIMMUNE INC [US]) 16 September 1999 (1999-09-16) * page 94, line 15 * * page 2, paragraph 4 * * page 3, paragraph 6 * * page 7, paragraph 1 * * page 11, paragraph 4 * * page 17, last paragraph - page 18, paragraph 1 * * page 18, paragraph 3 * ----- | 12,14-16 | |
| | | | TECHNICAL FIELDS SEARCHED (IPC) |

~~The present search report has been drawn up for all claims~~

4

| Place of search | Date of completion of the search | Examiner |
|---|---|---|
| The Hague | 10 December 2018 | Noë, Veerle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

Europäisches Patentamt
European Patent Office
Office européen des brevets

Application Number

EP 18 16 4740

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-4(completely); 5-16(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 18 16 4740

The Search Division considers that the present European patent application does not comply with the requirements of unity of invention and relates to several inventions or groups of inventions, namely:

1. claims: 4(completely); 1-3, 5-10, 16(partially)

   Method for producing a cellular tumor vaccine comprising antigen presenting cells comprising one or more genetic expression constructs for a major Histocompatibility Complex class I fusion protein comprising an N-terminal MHC class I antigenic peptide, a beta2 microglobulin and a C-terminal membrane anchor; the cellular composition and the medical use.

---

2. claims: 11(completely); 1-3, 5-10, 13-16(partially)

   Method for producing a cellular tumor vaccine comprising antigen presenting cells comprising one or more genetic expression constructs for a chimeric invariant chain wherein the CLIP sequence is replaced by a sequence comprising an MHC class II antigenic peptide; the cellular composition; a nucleic acid comprising a nucleotide sequence of the genetic expression construct; a chimeric CD74 protein, a nucleic acid encoding it and a host cell comprising the chimeric CD74 protein or its nucleic acid and the medical use.

---

3. claims: 12-16(partially)

   An antigenic peptide comprising a sequence according to SEQ ID No:3-14.

---

# ANNEX TO THE EUROPEAN SEARCH REPORT ON EUROPEAN PATENT APPLICATION NO. EP 18 16 4740

This annex lists the patent family members relating to the patent documents cited in the above-mentioned European search report. The members are as contained in the European Patent Office EDP file on
The European Patent Office is in no way liable for these particulars which are merely given for the purpose of information.

10-12-2018

| Patent document cited in search report | Publication date | Patent family member(s) | Publication date |
|---|---|---|---|
| WO 2008041231 A2 | 10-04-2008 | EP 2066690 A2 | 10-06-2009 |
| | | US 2008286312 A1 | 20-11-2008 |
| | | WO 2008041231 A2 | 10-04-2008 |
| US 2007055049 A1 | 08-03-2007 | US 2007055049 A1 | 08-03-2007 |
| | | US 2008260762 A1 | 23-10-2008 |
| WO 2004031211 A2 | 15-04-2004 | AU 2003291632 A1 | 23-04-2004 |
| | | CA 2500715 A1 | 15-04-2004 |
| | | EP 1578432 A2 | 28-09-2005 |
| | | JP 2006512300 A | 13-04-2006 |
| | | US 2006079453 A1 | 13-04-2006 |
| | | WO 2004031211 A2 | 15-04-2004 |
| WO 9945954 A1 | 16-09-1999 | AU 6465598 A | 27-09-1999 |
| | | CA 2323632 A1 | 16-09-1999 |
| | | EP 1064022 A1 | 03-01-2001 |
| | | JP 2002507397 A | 12-03-2002 |
| | | WO 9945954 A1 | 16-09-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3989816 A
- US 4444762 A


### Non-patent literature cited in the description


- **CAFRI G et al.** *Ann N Y Acad Sci,* 2013, vol. 1283, 87-90
- **CAFRI G et al.** *Int Immuno,* 2011, vol. 23, 453-61
- **SHARBI-YUNGER A et al.** *Oncoimmunology,* 2016
- **KUBY.** Immunology. W.H. Freeman and company, 2000
- **CODING, J. W.** Monoclonal Antibodies: Principles and Practice. Academic Press, 1986, 65-66, 71-74
- Monoclonal Antibody Technology. **CAMPBELL.** Laboratory Techniques in Biochemistry and Molecular Biology. Elseview, 1984, vol. 13, 75-83
- **KOHLER ; MILSTEIN.** *Nature,* 1975, vol. 256, 495-497
- **KOHLER ; MILSTEIN.** *Eur. J. Immunol.,* 1976, vol. 6, 511-519
- **GEFTER et al.** *Somatic Cell Genet,* 1977, vol. 3, 231-236
- **STEINMAN et al.** *Ann. Rev. Immunol.,* 1991, vol. 9, 271
- **CAFRI G et al.** mRNA-transfected dendritic cells expressing polypeptides which link MHC-I presentation to constitutive TLR4 activation confer tumor immunity. *Mol Ther,* 2015
- **GRAY KA ; YATES B ; SEAL RL ; WRIGHT MW ; BRUFORD EA.** genenames.org: the HGNC resources in 2015. *Nucleic Acids Res.,* January 2015, vol. 43, 1079-85
- **RAUSCH et al.** *J Immunol,* 2010, 113-126
- **DOUGAN et al.** *Cancer Immunol Res,* 2013